# EUROPÄISCHE PATENTSCHRIFT

(11) **EP 2 283 790 B1**
(45) Veröffentlichungstag und Bekanntmachung des Hinweises auf die Patenterteilung: **08.10.2014**
(21) Anmeldenummer: 10008373.2
(22) Anmeldetag: 11.08.2010
(51) Int. Cl.: A61B 19/00, F21W 131/205, A61B 17/00

(54) **Steuerung und Verfahren zum Betreiben einer Operationsleuchte**
Control and method for operating an operation light
Commande et procédé de fonctionnement d'une lampe d'opération

(30) Priorität: 14.08.2009 DE 102009037316
(43) Veröffentlichungstag der Anmeldung: 16.02.2011
(73) Patentinhaber: Karl Storz GmbH & Co. KG, 78532 Tuttlingen (DE)
(72) Erfinder: Hiltl, Christoph, 78532 Tuttlingen (DE); Abri, Omid, 14129 Berlin (DE); Schrader, Stefan, 14532 Kleinmachnow (DE)
(74) Vertreter: Fugmann, Winfried

(56) Entgegenhaltungen:
- EP-A1- 1 728 482
- EP-A1- 2 215 987
- WO-A2-2008/042219
- CN-Y- 201 248 169
- DE-A1- 19 845 027
- US-A- 4 639 838
- US-A1- 2008 242 993

## Beschreibung

Die vorliegende Erfindung bezieht sich auf eine Steuerung zum Steuern einer Operationsleuchte, ein Verfahren zum Betreiben einer Operationsleuchte, eine Operationsleuchte, ein Computer-Programm und eine Steuerungseinrichtung.

Viele diagnostische oder therapeutische Tätigkeiten setzen voraus oder beruhen auf einer präzisen optischen Erfassung eines Operationsfelds oder eines anderen Bereichs durch medizinisches Personal. Dazu ist eine helle und blendfreie Ausleuchtung erforderlich. Dies gilt beispielsweise für invasive oder mikroinvasive Operationen mit therapeutischem und/oder diagnostischem Zweck, für dermatologische Untersuchungen sowie zahnmedizinische, kieferorthopädische und kieferchirurgische Untersuchungen und Eingriffe.

Da in der Regel das Raumlicht bzw. Umgebungslicht für eine optimale Ausleuchtung nicht ausreicht, werden Leuchten verwendet, die im Folgenden unabhängig von ihrem vorgesehenen Einsatzort innerhalb eines Operationssaals oder an einem anderen Ort als Operationsleuchten bezeichnet werden. Die vorliegende Erfindung bezieht sich jedoch insbesondere auf Operationsleuchten, die für einen Einsatz innerhalb eines Operationssaals vorgesehen sind, da dort spezifische Probleme in besonderem Ausmaß und in besonderer Weise auftreten, die zur vorliegenden Erfindung geführt haben, und da Vorteile der vorliegenden Erfindung dort in besonderer Weise und in besonderem Maße zum Tragen kommen. Deshalb wird im Folgenden auch insbesondere auf die Situation im Operationssaal eingegangen.

Für eine optimale Ausleuchtung werden vor einem Eingriff eine oder mehrere Operationsleuchten manuell auf das Operationsgebiet eingestellt. Während der Operation erfolgt erforderlichenfalls eine Nachjustierung. Dazu ist an jeder Operationsleuchte ein sterilisierbarer Griff angeordnet.

In der DE 198 11 984 A1 ist ein Signalübertragungsnetzwerk beschrieben, an das Kameras, fernsteuerbare Operationsleuchten und Steuergeräte angeschlossen sind.

In der WO 2007/054367 A1 ist ein Operationsleuchtensystem mit mehreren Operationsleuchten mit je einer Steuerung beschrieben. Die Steuerungen der Operationsleuchten kommunizieren über Datenschnittstellen und Datenleitungen miteinander.

In der DE 42 01 934 A1 wird die Steuerung eines Datenverarbeitungssystems durch Gestik vorgeschlagen.

In der DE 200 01 134 U1 ist ein Operationssystem beschrieben, das eine Projektionsfläche zum Projizieren von Bedienelementen auf eine Projektionsfläche und eine Detektionseinrichtung zur Erkennung der Bewegung eines Fingers auf der Projektionsfläche umfasst.

In der DE 10 2008 019 191 A1 ist eine Vorrichtung zur gleichmäßigen Ausleuchtung eines Operationsfelds beschrieben. Eine Kamera erfasst ein Bild des Operationsfelds. Einzelne Leuchtelemente einer Operationsleuchte werden geschaltet, um eine gleichmäßige Ausleuchtung zu erzielen.

In der EP 1 728 482 A1, welche als Basis für die zweiteilige Form des nachfolgenden Anspruchs 1 herangezogen wurde, ist ein selbsteinstellendes Operationslampensystem beschrieben. In Gelenken eines Arms einer beweglichen Halterung einer Operationslampe sind Drehstellmotoren vorgesehen. Bilder einer Kamera werden verwendet, um die Position der Lampe zu steuern bzw. zu regeln, wenn das Sichtfeld der Kamera eingeschränkt ist.

In der DE 198 45 028 A1 ist ein Magnetresonanz-System beschrieben, das mittels Gesten, die von einer Kamera erfasst werden, gesteuert wird.

In der DE 198 03 494 A1 ist eine Operationsleuchte mit einer im Handgriff des Leuchtenkörpers angeordneten Miniaturkamera beschrieben. Bildsignale werden entlang des Arms der Operationsleuchte übertragen.

In der DE 20 2007 007 054 U1 ist ein System mit einer Operationsleuchte und einer Kamera beschrieben. Für einen automatischen Weißabgleich Informationen über die gewählte Farbtemperatur der Operationsleuchte an die Kamera übertragen In der US 2005/0232467 A1 ist eine Vorrichtung zum Erfassen einer Bewegung eines Objekts, insbesondere einer Hand, mittels eines Trägheitssensors und einer an dem Trägheitssensor befestigten Kamera zum Erfassen eines Bilds der Umgebung des Objekts beschrieben.

In der EP 1 408 443 A1 sind ein Verfahren und eine Vorrichtung zum Analysieren von Gesten einer Person zum Steuern einer Medienwiedergabevorrichtung mittels Gestenerkennung beschrieben.

In der WO 00/69354 A1 ist ein steuerbarer Kameraträger für Anwendungen in der Telemedizin beschrieben. Der steuerbare Kameraträger soll Kollisionen oder Kontakte mit medizinischer Ausrüstung, Operationslampen und medizinischem Personal vermeiden. Ferner ist ein Algorithmus beschrieben, um die Kamera zu bewegen, wenn die Sicht auf das betrachtete Objekt unterbrochen ist.

In der DE 1 097 382 ist eine Operationsleuchte mit einer lichtelektrischen Steuerung zur Bewegung der Operationsleuchte beschrieben. Für eine Verstellung in einer Richtung sind zwei Fotozellen im Gehäuse der Operationsleuchte mit nachgeschalteten Verstärkern vorgesehen. Ein Operateur kann mit einem Lichtgeber, der beispielsweise UV- und/oder moduliertes Licht aussendet, die Operationsleuchte steuern. Wenn beide Fotozellen unterschiedlich viel Licht von dem Lichtgeber empfangen, wird über ein Relais ein Motor gesteuert, der die Operationsleuchte nachführt bis beide Fotozellen wieder gleich viel Licht empfangen.

In der nachveröffentlichten EP 2 215 987 A1 ist eine Vorrichtung zur Beleuchtung eines Operationsfeldes eine sterilen Operationsraumes beschrieben. Ein Operateur kann eine Justierhilfe, die eine Einhandbedienung ermöglicht, in den Bereich bewegen, der beleuchtet werden soll. Eine Positions-Erfassungseinrichtung erfasst die Position der Justierhilfe und richtet eine an ei-nem Schwenkarm bewegliche Leuchte auf die Justierhilfe aus. Zur Erfassung der Position der Justierhilfe sind Kameras und eine Bilderkennungssoftware in einer Auswerte- und Steuereinrichtung vorgesehen.

In der WO 2008/042219 A2 ist eine Steuerung medizinischer Geräte mittels Gesten beschrieben. Die Gesten werden mittels einer Kamera oder mittels eines "tracking glove" erfasst.

In der DE 198 45 027 A1 ist ein medizintechnisches System beschrieben, bei dem Bedienelemente auf eine Projektionsfläche projiziert werden. Mittels einer Detektoreinrichtung, insbesondere einer Videokamera, wird die Gestik einer Bedienperson erfasst.

Diese Operationsleuchte weist eine Reihe von Nachteilen auf. Unter anderem besteht ein Nachteil dieser Operationsleuchte für viele Anwendungen darin, dass der Lichtgeber von Hand bedient werden muss.

Eine Aufgabe der vorliegenden Erfindung besteht darin, eine verbesserte Steuerung zum Steuern einer Operationsleuchte durch medizinisches Personal, ein verbessertes Verfahren zum Betreiben einer Operationsleuchte, eine verbesserte Operationsleuchte, ein verbessertes Computer-Programm und eine verbesserte Steuerungseinrichtung zu schaffen.

Diese Aufgabe wird durch die Gegenstände der unabhängigen Ansprüche gelöst.

Weiterbildungen sind in den abhängigen Ansprüchen angegeben.

Verschiedene Ausführungsformen der vorliegenden Erfindung beruhen auf der Idee, eine Operationsleuchte mit einer Kamera zum Erfassen eines Bilds eines Operationsfelds, einem Bildsignalausgang und einem Steuersignaleingang so auszubilden, dass zumindest entweder eine Richtung oder eine spektrale Eigenschaft des Lichts oder eine Größe oder Gestalt des von dem Licht beleuchteten Bereichs in Abhängigkeit von einem Steuersignal veränderbar bzw. mittels des Steuersignals steuerbar sind. Ein Bildsignaleingang einer Steuerung ist mit dem Bildsignalausgang der Operationsleuchte gekoppelt, ein Steuersignalausgang der Steuerung ist mit dem Steuersignaleingang der Operationsleuchte gekoppelt. Die Steuerung ist ausgebildet, um das Steuersignal in Abhängigkeit von dem Bildsignal zu erzeugen und am Steuersignalausgang auszugeben. Von der Kamera wird ein Bild des Operationsfelds erfasst und ein Bildsignal erzeugt. Das Bildsignal wird von der Kamera zu einer Steuerung übertragen. In Abhängigkeit von dem Bildsignal wird von der Steuerung ein Steuersignal erzeugt. Dabei wird insbesondere eine Position, eine Bewegung oder eine Geste eines Handbereichs, insbesondere einer Hand, eines Operateurs oder von anderem medizinischem Personal anhand des Bildsignals erfasst und geht in die Erzeugung des Steuersignals ein.

Insbesondere bei großflächigen Operationsfeldern, beispielsweise bei Unfallopfern, schafft die vorliegende Erfindung eine deutliche Vereinfachung und Verbesserung der Handhabung. Das medizinische Personal kann sich in stärkerem Maße auf seine eigentliche Aufgabe konzentrieren, da es sich nicht mehr mit einer Verstellung der Operationsleuchte befassen muss. Insbesondere folgt der von der Operationsleuchte erzeugte Lichtkegel den Handlungen des Operateurs oder von anderem medizinischem Personal und/oder wird von der Steuerung hinsichtlich seiner Größe und Ausdehnung an das Operationsfeld angepasst.

Eine Steuerung zum Steuern einer Operationsleuchte zum Erzeugen von Licht - insbesondere zur Beleuchtung eines Operationsfelds - umfasst einen Bildsignaleingang zum Empfangen eines Bildsignals von einer Kamera zum Erfassen eines Bilds des Operationsfelds und einen Steuersignalausgang zum Ausgeben eines Steuersignals zur Steuerung zumindest entweder einer Richtung oder einer spektralen Eigenschaft des Lichts oder einer Größe oder Gestalt des von dem Licht zu beleuchtenden Bereichs an die Operationsleuchte. Die Steuerung ist ausgebildet, um einen Handbereich von medizinischem Personal in einem von der Kamera empfangenen Bildsignal zu erkennen und das Steuersignal in Abhängig von dem Bildsignal zu erzeugen und am Steuersignalausgang auszugeben.

Der Handbereich des medizinischen Personals umfasst insbesondere eine Hand und/oder einen daran anschließenden Körperbereich, insbesondere einen Unterarm, einen Handschuh, ein Armband, einen Ärmel oder eine andere Bekleidung. Die Steuerung kann ausgebildet sein, um den Handbereich anhand seiner Gestalt, anhand seiner Farbe oder anhand einer Markierung an der Hand, am Handschuh, an einem Armband, an einem Ärmel oder an einem anderen Ort zu erkennen.

Die Steuerung ist ausgebildet, um anhand des Bildsignals eine Geste eines Handbereichs von medizinischem Personal oder einer Markierung im Handbereich des medizinischen Personals zu erkennen bzw. zu erfassen und das Steuersignal in Abhängigkeit von der Position bzw. der Bewegung bzw. der Geste zu erzeugen.

Abhängig von der vorgesehenen Anwendung kann die Erkennung einer Markierung an einem Handbereich, insbesondere an einer Hand oder einem Arm, des medizinischen Personals mittels einer in die Steuerung integrierten Objekterkennung eine besonders einfach realisierbare und im Betrieb besonders robuste Option sein. Zu diesem Zweck unterscheidet sich die Markierung beispielsweise in ihrer Form, in ihren Reflexionseigenschaften, durch eine Fluoreszenz oder auf andere Weise von anderen durch die Kamera erfassten Objekten.

Alternativ oder zusätzlich ist die Steuerung ausgebildet, um die Position oder die Positionen des medizinischen Personals oder weiterer Körperteile des medizinischen Personals zu erfassen. Beispielsweise können die Positionen der Arme oder des Kopfes eines Operateurs oder anderen medizinischen Personals erfasst werden, um in Abhängigkeit von diesen Positionen das Steuersignal so zu erzeugen, dass eine Abschattung des von der Operationsleuchte erzeugten Lichts verhindert wird. Beispielsweise wird die Operationsleuchte in Abhängigkeit von der Position oder den Positionen der Köpfe und der Arme des medizinischen Personals verfahren. Bei einer Operationsleuchte, die mehrere Einzelleuchten umfasst, können alternativ Einzelleuchten, deren Licht durch medizinisches Personal abgeschattet wird, abgeschaltet und andere Einzelleuchten zugeschaltet werden, um die Beleuchtungsleistung aufrechtzuerhalten.

Alternativ oder zusätzlich ist die Steuerung ausgebildet, um Position und Ausdehnung des Operationsfelds, insbesondere die Ränder des Operationsfelds, zu erkennen und das Steuersignal in Abhängigkeit von der Position oder Ausdehnung des Operationsfelds oder der Lage seiner Ränder zu erzeugen.

Die Steuerung kann ferner ausgebildet sein, um die Position oder eine Bewegung oder eine Geste eines mit einer Markierung versehenen Handbereichs von medizinischem Personal anhand eines Sensorsignals eines an die Markierung angepassten Sensors zu erkennen und das Steuersignal in Abhängigkeit von der mittels des Sensorsignals ermittelten Position bzw. Bewegung bzw. Geste zu erzeugen.

Die Steuerung kann ausgebildet sein, um anhand des Bildsignals zu bestimmen, ob eine Position eines Handbereichs von medizinischem Personal in einem vorbestimmten Bereich liegt, insbesondere ob sie innerhalb des Operationsfelds liegt, und um das Steuersignal in Abhängigkeit davon zu erzeugen, ob die Position des Handbereichs in dem vorbestimmten Raumbereich liegt.

Ferner kann die Steuerung ausgebildet sein, um eine Geschwindigkeit eines Handbereichs von medizinischem Personal zu bestimmen, um zu bestimmen, ob die Geschwindigkeit des Handbereichs des medizinischen Personals in einem vorbestimmten Geschwindigkeitsbereich liegt, und um das Steuersignal in Abhängigkeit davon zu erzeugen, ob die Geschwindigkeit des Handbereichs des medizinischen Personals in dem vorbestimmten Geschwindigkeitsbereich liegt.

Beispielsweise ist die Steuerung so ausgebildet, dass die Position, die Bewegung oder eine Geste einer Hand eines medizinischen Personals ignoriert wird, wenn gleichzeitig die Position der Hand oder die Geschwindigkeit der Hand außerhalb eines vorbestimmten Bereichs liegt.

Die Steuerung kann ferner ausgebildet sein, um ein Positionierungssignal zu empfangen, das eine Position oder eine Veränderung einer Position eines verstellbaren Operationstischs oder eines Gegenstands, mit dem die Operationsleuchte kollidieren kann, darstellt, und um das Steuersignal in Abhängigkeit von dem Positionierungssignal zu erzeugen.

Dieser Gegenstand ist beispielsweise eine weitere Operationsleuchte, ein Bildschirm oder ein Geräteträger, der feststehend oder bewegbar an der Decke des Operationssaals angebracht ist oder auf dem Boden des Operationssaals steht. Das Positionierungssignal kann ein Soll-Signal sein, das eine anzufahrende Position oder eine vorzunehmende Veränderung der Position des verstellbaren Operationstischs oder des Gegenstands darstellt und beispielsweise zur Steuerung des Operationstischs oder des Gegenstands - sofern dieser bewegbar ist - verwendet wird. Alternativ oder zusätzlich kann das Positionierungssignal ein Ist-Signal sein, das eine tatsächlich vorliegende Position oder eine tatsächlich vorliegende Veränderung der Position des verstellbaren Operationstischs bzw. des Gegenstands darstellt. Die Steuerung ist beispielsweise ausgebildet, um das Positionierungssignal über einen Bus oder eine andere Signalleitung von einem Sensor oder einer anderen Einrichtung oder von einem Speicher zu empfangen. Der Speicher und die Steuerung können in einem gemeinsamen Gehäuse angeordnet sein. In dem Speicher ist beispielsweise die Position des Gegenstands abgelegt, mit dem die Operationsleuchte kollidieren kann. Das Positionierungssignal kann in die Erzeugung des Steuersignals eingehen, um beispielsweise bei einer Veränderung der Position des verstellbaren Operationstischs eine gleichzeitige Nachführung der Operationsleuchte zu steuern oder um eine Kollision der Operationsleuchte mit dem Gegenstand zu vermeiden.

Alternativ oder zusätzlich zur Erkennung eines Handbereichs kann die Steuerung ausgebildet sein, um zumindest entweder eine Bewegung eines Kopfs, eine Mimik eines Gesichts oder eine Augenbewegung von medizinischem Personal zu erkennen und das Steuersignal in Abhängigkeit von der Bewegung des Kopfs, der Mimik des Gesichts oder der Augenbewegung zu erzeugen.

Insbesondere ist die Steuerung ausgebildet, um einen Kopf in einem von der Kamera empfangenen Bildsignal zu erkennen, eine Bewegung des Kopfs zu erkennen und das Steuersignal in Abhängigkeit von der erkannten Bewegung des Kopfs zu erzeugen. Dazu identifiziert die Steuerung vorbestimmte Bewegungsmuster als Befehle zur Steuerung der Operationsleuchte.

Alternativ oder zusätzlich kann die Steuerung ausgebildet sein, um ein Gesicht in einem von der Kamera empfangenen Bildsignal zu erkennen, eine Mimik des Gesichts zu erkennen und das Steuersignal in Abhängigkeit von der erkannten Mimik zu erzeugen. Dazu identifiziert die Steuerung vorbestimmte Mimikmuster als Befehle zur Steuerung der Operationsleuchte.

Alternativ oder zusätzlich kann die Steuerung ausgebildet sein, um ein Auge oder beide Augen in einem von der Kamera empfangenen Bildsignal zu erkennen, eine Blickrichtung, eine Änderung der Blickrichtung, ein Blinzeln und/oder eine andere Augenbewegung zu erkennen und das Steuersignal in Abhängigkeit von der erkannten Augenbewegung zu erzeugen. Dazu identifiziert die Steuerung vorbestimmte Augenbewegungen als Befehle zur Steuerung der Operationsleuchte.

Zur Steuerung der Operationsleuchte mittels Augenbewegungen kann eine graphische Benutzerschnittstelle (engl.: graphical user interface, GUI) auf einem Bildschirm dargestellt oder an eine Fläche projiziert werden, der bzw. die im Blickbereich des medizinischen Personals liegt. Diese graphische Benutzerschnittstelle kann Bedienknöpfe und Schaltflächen umfassen, denen Steuerfunktionen zugeordnet sind. Zu diesen Steuerfunktionen zählen beispielsweise das Dimmen der Operationsleuchte oder einer der Operationsleuchten oder von Einzelleuchten der Operationsleuchte oder der Allgemeinbeleuchtung ("heller"/"dunkler"); das Ändern der Position der Operationsleuchte oder einer der Operationsleuchten oder einer Einzelleuchte der Operationsleuchte ("links"/"rechts", "vor"/"zurück", "rauf"/"runter"); das Ändern der Fokussierung bzw. der Gestalt und/oder der Größe des beleuchteten Bereichs ("größer"/"kleiner", "schmäler"/"breiter", "länger"/"kürzer"); das Ändern der Farbtemperatur der Operationsleuchte und/oder der Allgemeinbeleuchtung ("wärmer"/"kälter" bzw. "tiefer"/"höher").

Die Steuerung ist beispielsweise ausgebildet, um zu erkennen, dass der Blick des relevanten medizinischen Personals auf einer Schaltfläche ruht und von dieser langsam nach oben oder nach unten gleitet, oder um zu erkennen, dass der Blick auf einer Schaltfläche ruht, und gleichzeitig ein Blinzeln zu erkennen, und diese Augenbewegungen mit einer Betätigung der Schaltfläche zu identifizieren, oder um zu erkennen, dass die Verweildauer (engl.: dwell time) des Blicks auf einer Schaltfläche einen ersten vorbestimmten Schwellenwert überschreitet und/oder einen zweiten vorbestimmten Schwellenwert unterschreitet.

Alternativ oder zusätzlich zu Schaltflächen kann die graphische Benutzerschnittstelle eine realitätsnahe oder schematisierte Abbildung des Operationstischs und/oder des Patienten und/oder des Operationsfelds zeigen. Die Steuerung ist beispielsweise ausgebildet, um durch Augenbewegungen das Licht der Operationsleuchte an einen bestimmten Ort zu steuern, auf dessen Repräsentation in der Abbildung der Blick des medizinischen Personals ruht, oder um aufgrund einer Augenbewegung, die den auszuleuchtenden Bereich umschreibt, die Fokussierung der Operationsleuchte einzustellen.

Ein Operationsleuchtensystem umfasst eine Steuerung, wie sie hier beschrieben ist, und eine Operationsleuchte zum Erzeugen von Licht, wobei die Operationslechte eine Kamera zum Erfassen eines Bilds eines Operationsfelds und zum Erzeugen eines Bildsignals, einen Bildsignalausgang zum Ausgeben des Bildsignals an eine Steuerung und einen Steuersignaleingang zum Empfangen eines Steuersignals von der Steuerung umfasst. Die Operationsleuchte ist ausgebildet, um zumindest entweder eine Richtung oder eine Intensität oder eine spektrale Eigenschaft des Lichts oder eine Größe oder Gestalt des von dem Licht beleuchteten Bereichs in Abhängigkeit von dem Steuersignal zu verändern.

Bei dem Operationsleuchtensystem kann die Operationsleuchte zumindest entweder an einer Schieneneinrichtung angeordnet sein oder eine Mehrzahl von Einzelleuchten umfassen, die in einem Array oder auf andere gleichförmige oder ungleichförmige Art angeordnet sind.

Ein Operationsleuchtensystem umfasst eine Operationsleuchte zum Erzeugen von Licht und eine Schieneneinrichtung, wobei die Operationsleuchte entlang der Schieneneinrichtung verfahrbar ist.

Die Operationsleuchte kann eine Operationsleuchte, wie sie oben beschrieben ist, sein, also insbesondere eine Kamera umfassen. Das Operationsleuchtensystem kann ferner eine Steuerung umfassen, wie sie oben beschrieben ist. Das Operationsleuchtensystem kann mehrere Operationsleuchten umfassen, welche miteinander gekoppelt oder unabhängig voneinander entlang der Schieneneinrichtung verfahrbar sind. Die Operationsleuchte oder die Operationsleuchten sind insbesondere aufgrund von Steuersignalen verfahrbar, die von der oben beschriebenen Steuerung erzeugt werden.

Die Schieneneinrichtung des Operationsleuchtensystems kann so ausgebildet sein, dass das gesamte Operationsleuchtensystem eine geringe Bauhöhe aufweist. Dadurch kann das Operationsleuchtensystem auch in einem niedrigen Operationssaal eingesetzt werden, in dem die Verwendung eines herkömmlichen Leuchtenarms mit mehreren Gelenken nachteilig ist, weil die Operationsleuchte zu tief hängt und/oder Bewegungen des medizinischen Personals einschränkt. Auch in einem Operationssaal mit gewöhnlicher Raumhöhe kann eine geringe Bauhöhe vorteilhaft sein, weil die Operationsleuchte in größerer Höhe angeordnet sein kann und damit das medizinische Personal weniger oder gar nicht behindert. Ferner können weitere Vorrichtungen, beispielsweise Bildschirme oder andere Geräte, freier positioniert werden, wenn die Operationsleuchte in größerer Höhe angeordnet ist.

Die Schieneneinrichtung umfasst insbesondere eine einfache Schiene oder zwei oder mehr parallele Schienen. An dieser Schiene bzw. diesen Schienen ist eine Laufkatzeneinrichtung verfahrbar angeordnet. Die Laufkatzeneinrichtung ist teilweise oder vollständig über der Schiene bzw. über den Schienen angeordnet. Alternativ ist die Laufkatzeneinrichtung unter der Schiene bzw. den Schienen angeordnet und umgreift diese teilweise oder vollständig. Die Laufkatzeneinrichtung ist insbesondere elektromotorisch angetrieben, um aufgrund eines Steuersignals entlang der Schiene bzw. der Schienen verfahren zu werden.

Die Schiene bzw. die Schienen können eine kreisförmige, elliptische, ovale, im Wesentlichen rechteckige, U-förmige, L-förmige oder beliebige andere Gestalt aufweisen, um eine entsprechende Verfahrbarkeit der Operationsleuchte zu ermöglichen. Insbesondere umfasst die Schieneneinrichtung eine erste und eine zweite voneinander beabstandete parallele Schienen, an denen ähnlich wie bei einem Portalkran eine dritte, quer zur ersten und zur zweiten Schiene angeordnete Schiene verfahrbar ist, wobei die Operationsleuchte entlang der dritten Schiene verfahrbar ist.

Die Operationsleuchte kann an der Laufkatzeneinrichtung starr oder um eine oder mehrere Schwenkachsen schwenkbar angeordnet sein. Ferner kann die Operationsleuchte gegenüber der Laufkatzeneinrichtung translatorisch bewegbar sein. Beispielsweise ist die Operationsleuchte entlang einer an der Laufkatzeneinrichtung starr oder bewegbar angeordneten Schiene verfahrbar oder an einem schwenkbaren Arm angeordnet.

Bei einem Verfahren zum Betreiben einer Operationsleuchte zum Erzeugen von Licht, insbesondere zum Beleuchten eines Operationsfelds, werden mittels einer Kamera ein Bild des Operationsfelds erfasst und ein Bildsignal erzeugt, das Bildsignal von der Kamera zu einer Steuerung übertragen, ein Handbereich von medizinischem Personal erkannt, in Abhängigkeit von dem Bildsignal ein Steuersignal erzeugt und zumindest entweder eine Richtung oder eine spektrale Eigenschaft des Lichts oder eine Größe oder Gestalt des von dem Licht beleuchteten Bereichs mittels des Steuersignals gesteuert.

Der Handbereich des medizinischen Personals umfasst insbesondere eine Hand oder einen daran anschließenden Körperbereich, insbesondere einen Unterarm, einen Handschuh, ein Armband, einen Ärmel oder eine andere Bekleidung. Bei dem Verfahren kann der Handbereich anhand seiner Gestalt, anhand seiner Farbe oder anhand einer Markierung an der Hand, am Handschuh, an einem Armband, an einem Ärmel oder an einem anderen Ort erkannt werden.

Bei dem Verfahren kann ferner zumindest entweder eine Position oder eine Bewegung oder eine Geste eines Handbereichs von medizinischem Personal oder einer Markierung im Handbereich des medizinischen Personals anhand des Bildsignals bestimmt werden, wobei das Steuersignal in Abhängigkeit von der Position bzw. der Bewegung bzw. der Geste des Handbereichs erzeugt wird.

Abhängig von der vorgesehenen Anwendung kann die Erkennung einer Markierung in einem Handbereich des medizinischen Personals mittels eines Algorithmus zur Objekterkennung eine besonders einfach realisierbare und im Betrieb besonders robuste Option sein. Zu diesem Zweck unterscheidet sich die Markierung beispielsweise in ihrer Form, in ihren Reflexionseigenschaften, durch eine Fluoreszenz oder auf andere Weise von anderen durch die Kamera erfassten Objekten.

Bei dem Verfahren kann ferner zumindest entweder eine Position oder eine Bewegung oder eine Geste eines mit einer Markierung versehenen Handbereichs von medizinischem Personal anhand eines Sensorsignals eines an die Markierung angepassten Sensors bestimmt werden, wobei das Steuersignal ferner in Abhängigkeit von der anhand des Sensorsignals bestimmten Position bzw. Bewegung bzw. Geste des Handbereichs des medizinischen Personals erzeugt wird.

Ferner kann bestimmt werden, ob die Position des Handbereichs, insbesondere der Markierung, in einem vorbestimmten Raumbereich liegt, wobei das Steuersignal ferner in Abhängigkeit davon erzeugt wird, ob die Position des Handbereichs in dem vorbestimmten Raumbereich liegt.

Ferner kann die Geschwindigkeit des Handbereichs des medizinischen Personals bestimmt werden und bestimmt werden, ob die Geschwindigkeit in einem vorbestimmten Geschwindigkeitsbereich liegt, wobei das Steuersignal in Abhängigkeit davon erzeugt wird, ob die Geschwindigkeit in dem vorbestimmten Geschwindigkeitsbereich liegt.

Beispielsweise wird eine Position oder eine Bewegung oder eine Geste eines Handbereichs, insbesondere einer Hand, des medizinischen Personals bzw. einer Markierung im Handbereich ignoriert und hat keinen Einfluss auf die Erzeugung des Steuersignals, wenn die Position des Handbereichs bzw. der Markierung außerhalb des vorbestimmten Bereichs liegt oder der Handbereich bzw. die Markierung zu schnell bewegt wird.

Bei einem der oben beschriebenen Verfahren kann ferner ein Positionierungssignal erfasst werden, das eine Position oder eine Veränderung der Position eines verstellbaren Operationstischs oder eines Gegenstands, mit dem die Operationsleuchte kollidieren kann, anzeigt, wobei das Steuersignal in Abhängigkeit von dem Positionierungssignal erzeugt wird.

Das Positionierungssignal kann über einen Bus oder eine andere Signalleitung von einem Sensor oder einer anderen Einrichtung oder von einem Speicher empfangen werden, wobei der Speicher und die Steuerung in einem gemeinsamen Gehäuse angeordnet sein können. Wie oben in Zusammenhang mit der Steuerung dargestellt, ist dieser Gegenstand beispielsweise eine weitere Operationsleuchte, ein Bildschirm oder ein Geräteträger, der feststehend oder bewegbar an der Decke des Operationssaals angebracht ist oder auf dem Boden des Operationssaals steht. Wie ebenfalls bereits oben dargestellt, kann das Positionierungssignal ein Ist-Signal oder ein Soll-Signal sein. Dadurch kann bei einer Veränderung der Position des verstellbaren Operationstischs eine gleichzeitige Nachführung der Operationsleuchte gesteuert und/oder eine Kollision der Operationsleuchte mit einem anderen Gegenstand verhindert werden.

Bei einem der oben beschriebenen Verfahren kann ferner eine Identität des medizinischen Personals mittels der Kamera oder auf andere Weise erfasst werden.

Die Identität des medizinischen Personals wird beispielsweise anhand einer elektrisch, magnetisch, elektromagnetisch oder mittels Ultraschall erfassbaren Markierung oder eines Transponders an einem Handschuh oder einem Armband des medizinischen Personals erfasst. Dazu wird die Markierung insbesondere mittels eines entsprechenden Sensors ausgelesen, beispielsweise mittels eines Ultraschallsensors oder eines RFID-Sensors. Die Markierung kann bei entsprechender Ausbildung gleichzeitig oder abwechselnd oder wahlweise optisch und auf andere Weise (elektrisch, magnetisch, elektromagnetisch, mittels Ultraschall etc.) ausgelesen werden. Durch die nicht-optische Erfassung der Markierung kann anstelle der Erfassung der Identität des medizinischen Personals oder zusätzlich zu dieser auch einer Bestimmung der Position der Markierung erfolgen.

Nach dem Erfassen der Identität des medizinischen Personals kann dieser in einem Operationssystem angemeldet werden.

Auf der Grundlage der Anmeldung können beispielsweise von dem medizinischen Personal bevorzugte Einstellungen an Geräten im Operationssaal oder eine Eintragung in einem Protokoll erfolgen.

Eine Steuerungseinrichtung zum Steuern einer Operationsleuchte umfasst eine elektromagnetisch oder mittels Ultraschall erfassbare Markierung und eine Befestigungseinrichtung zum Befestigen der Markierung an einer Hand von medizinischem Personal. Die Befestigungseinrichtung ist beispielsweise ein Handschuh oder ein Armband für medizinisches Personal. Die Markierung umfasst beispielsweise eine im Infrarot, im Sichtbaren oder im UV emittierende Lichtquelle, eine Ultraschallquelle, einen Transponder, einen Reflektor für elektromagnetische Strahlung oder einen Fluoreszenzfarbstoff.

Bei allen Ausführungsformen sind die von der Kamera erzeugten und zur Steuerung übertragenen Bildsignale und die von der Steuerung erzeugten und zur Operationsleuchte übertragenen Steuersignale jeweils beispielsweise elektrische oder optische Signale, die über entsprechende Leitungen übertragen werden.

Bei allen beschriebenen Ausführungsformen kann die Kamera zur Bereitstellung eines Schwarzweißbilds oder eines mehrfarbigen Bilds ausgebildet sein. Die Kamera kann lediglich im für das menschliche Auge sichtbaren Spektralbereich empfindlich sein. Alternativ oder zusätzlich kann die Kamera im nahen oder im fernen Infrarot empfindlich sein. Ferner kann die Kamera eine Stereokamera sein bzw. zwei einzelne Kameras umfassen, die von unterschiedlichen Aufnahmepositionen aus im Wesentlichen auf das gleiche Aufnahmegebiet gerichtet sind. Ferner kann die Kamera eine Time-of-Flight-Kamera sein, die auf der Grundlage der Laufzeit eines Lichtpulses von einer Lichtquelle zum Objekt und vom Objekt zur Kamera den Abstand der Kamera vom Objekt bestimmt oder eine Bestimmung dieses Abstands ermöglicht.

Die Operationsleuchte ist beispielsweise eine Operationsleuchte mit einem in mehreren Freiheitsgraden verstellbaren Arm. Dieser Arm ist ähnlich einem Roboterarm, wie er in zahlreichen Varianten in der industriellen Fertigung verwendet wird, in seinen Freiheitsgraden motorisch verstellbar. Alternativ umfasst die Operationsleuchte eine oder mehrere Arrays von Leuchtdioden oder anderen Lichtquellen bzw. Einzelleuchten mit gerichteter Emission, die nahe an der Deck des Operationssaals angeordnet oder in die Decke eingelassen sind. Diese Arrays oder einzelne Lichtquellen oder Gruppen von Lichtquellen innerhalb eines Arrays sind insbesondere einzeln schaltbar und/oder dimmbar bzw. in ihrer Lichtemission steuerbar, um die Richtung, aus der Licht auf das Operationsfeld geworfen wird, zu steuern. Ferner kann für jede einzelne Lichtquelle oder für jeweils eine Gruppe von Lichtquellen die Fokussierung bzw. die Divergenz des von eine einzelnen Lichtquelle oder von einer Gruppe von Lichtquellen ausgehenden Lichtbündels einstellbar sein. Ferner können die Arrays oder einzelne Lichtquellen oder Gruppen von Lichtquellen mittels Stellmotoren um ein, zwei oder mehr Achsen bewegbar sein, insbesondere schwenkbar.

Die vorliegende Erfindung ist als Verfahren oder als Computer-Programm mit Programmcode zur Durchführung oder Steuerung eines solchen Verfahrens, wenn das Computer-Programm auf einem Computer oder einem Prozessor abläuft, implementierbar. Ferner ist die Erfindung als Computer-Programm-Produkt mit auf einem maschinenlesbaren Träger (beispielsweise einem ROM-, PROM-, EPROM-, EEPROM- oder Flash-Speicher, einer CD-ROM, DVD, HD-DVD, Blue-Ray-Disc, Diskette oder Festplatte) oder in Form von Firmware gespeichertem Programmcode zur Durchführung von einem der genannten Verfahren, wenn das Computer-Programm-Produkt auf einem Computer, Rechner oder Prozessor abläuft, implementierbar. Ferner kann die vorliegende Erfindung als digitales Speichermedium (beispielsweise ROM-, PROM-, EPROM-, EEPROM- oder Flash-Speicher, CD-ROM, DVD, HD-DVD, Blue-Ray-Disc, Diskette oder Festplatte) mit elektronisch auslesbaren Steuersignalen, die so mit einem programmierbaren Computer- oder Prozessor-System zusammenwirken können, dass eines der beschriebenen Verfahren ausgeführt wird, implementiert werden.

Ferner kann die vorliegende Erfindung als Steuerung implementiert werden, wobei die Steuerung ausgebildet ist, um eines der beschriebenen Verfahren auszuführen, oder wobei die Steuerung ein Computer-Programm, ein Computer-Programm-Produkt oder ein digitales Speichermedium umfasst, wie sie im vorangehenden Absatz beschrieben wurden.

### Kurzbeschreibung der Figuren

Nachfolgend werden Ausführungsformen anhand der beigefügten Figuren näher erläutert. Es zeigen:
- Figur 1: eine schematische Darstellung eines Operationssaals;
- Figur 2: eine schematische Darstellung einer Steuerung;
- Figur 3: eine schematische Darstellung von Operationsleuchten an einer Schieneneinrichtung;
- Figur 4: eine schematische Darstellung von Operationsleuchten an einer Schieneneinrichtung;
- Figur 5: eine schematische Darstellung einer Operationsleuchte an einer Schieneneinrichtung;
- Figur 6: eine schematische Darstellung einer Operationsleuchte an einer Schieneneinrichtung;
- Figur 7: ein schematisches Flussdiagramm eines Verfahrens zum Betreiben einer Operationsleuchte.

### Beschreibung der Ausführungsformen

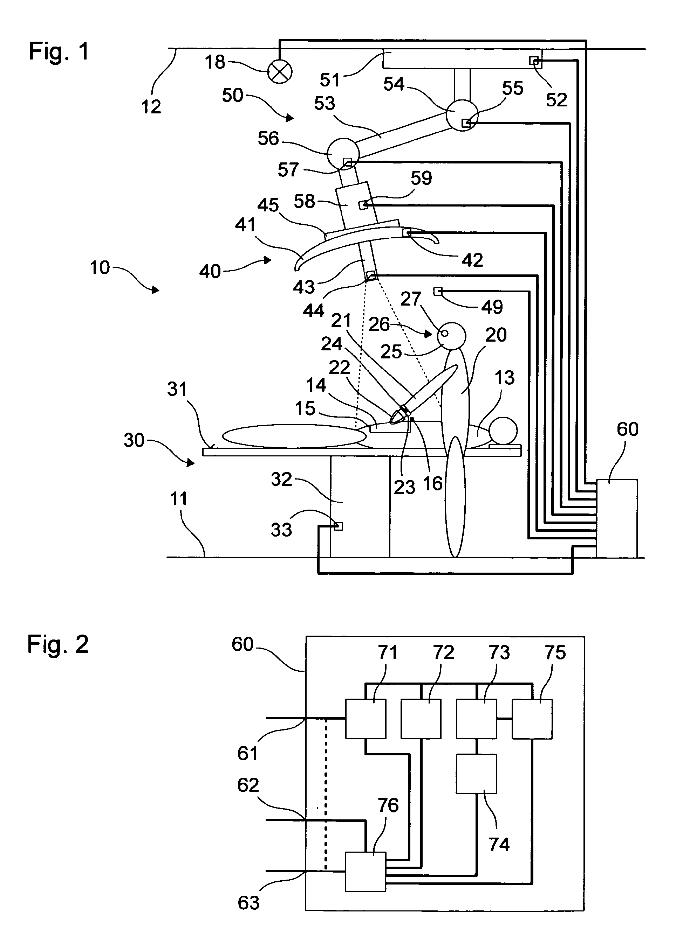

Figur 1 zeigt einen Operationssaal 10 mit einem Boden 11 und einer Decke 12. An einem Patienten 13 liegt ein Operationsfeld 14 mit einem Rand 15 vor. Das Operationsfeld 14 und sein Rand 15 sind beispielsweise durch die Art des diagnostischen oder therapeutischen Eingriffs definiert. Am Rand 15 des Operationsfelds 14 können eine oder mehrere elektrisch, magnetisch, elektromagnetisch oder mittels Ultraschall erfassbare Markierungen 16 vorgesehen sein. Der Operationssaal 10 weist eine Allgemeinbeleuchtung 18 auf, die insbesondere an der Decke 12 des Operationssaals 10 angeordnet ist, und die ausgebildet ist, um eine gleichmäßige Helligkeit im gesamten Operationssaals 10 bereitzustellen.

Ein Operateur 20 oder anderes medizinisches Personal trägt am distalen Ende seines Arms 21 bzw. an seiner Hand einen Handschuh 22 und/oder ein Armband 23 mit einer oder mehreren elektrisch, magnetisch, elektromagnetisch oder mittels Ultraschall erfassbaren Markierungen 24.

Der Patient 13 liegt auf einem Operationstisch 30 mit einer Liegefläche 31. Die Liegefläche 31 ist mittels eines Verstellantriebs 32 beispielsweise in der Höhe über dem Boden 11 und ihrer Neigung gegenüber dem Boden 11 verstellbar. Der Verstellantrieb 32 des Operationstischs 30 weist einen Steuersignaleingang 33 auf.

Über dem Operationsfeld 14 ist eine Operationsleuchte 40 mit einem Reflektor 41 und einem Steuersignalausgang 42 angeordnet. Die Operationsleuchte kann eine einzige Leuchte oder eine Mehrzahl von Einzelleuchten umfassen. Einzelleuchten der Operationsleuchte 40 umfassen beispielsweise je eine oder mehrere Leuchtdioden. Beispielsweise umfassen die Einzelleuchten je eine oder mehrere RGB-Leuchtdioden, RGBW-Leuchtdioden, auf Fluoreszenz oder Phosphoreszenz beruhende Weißlicht-Leuchtdioden, einzelne rote, grüne und blaue Leuchtdioden oder Leuchtdioden in Sekundärfarben. Jede einzelne Leuchtdiode kann eine anorganische oder eine organische Leuchtdiode (auch als OLED bezeichnet) sein.

Jede Einzelleuchte kann einen Reflektor umfassen, der verstellbar sein kann, um die Richtung und die Fokussierung bzw. die Divergenz des von der Einzelleuchte ausgehenden Lichtbündels zu verstellen. Einzelleuchten können zur Bereitstellung von Licht unterschiedlicher spektraler Eigenschaften, insbesondere Licht unterschiedlicher Wellenlängenbereiche oder Farben oder unterschiedlicher Farbtemperaturen ausgebildet sein. Das von der Operationsleuchte bereitgestellte Licht kann hinsichtlich seiner Richtung und der Ausdehnung des ausgeleuchteten Bereichs durch Zuschalten oder Abschalten von Einzelleuchten oder durch gemeinsames oder unabhängiges Schwenken von Einzelleuchten an den Ort und die Ausdehnung des Operationsfelds 14 angepasst werden.

Die Operationsleuchte 40 umfasst eine Kamera 43 mit einem Bildsignalausgang 44. Über einen mechanischen Adapter bzw. ein Trägersystem 45 sind der Reflektor 41 und die Kamera 43 der Operationsleuchte 40 mit einer Aufhängung 50 verbunden. Ferner sind im Operationssaal 10 einer oder mehrere Sensoren 49 angeordnet, beispielsweise Ultraschallsensoren oder RFID-Sensoren.

Die Aufhängung 50 der Operationsleuchte 40 umfasst einen an der Decke 12 des Operationssaals 10 befestigten Deckenträger 51 mit einem Steuersignaleingang 52. Ein Leuchtenarm 53 verbindet das Trägersystem 45 mit dem Deckenträger 51. An bzw. in dem Leuchtenarm 53 sind ein erstes Gelenk 54 mit einem Steuersignaleingang 55 und ein zweites Gelenk 56 mit einem Steuersignaleingang 57 vorgesehen. An dem dem Reflektor 41 zugewandten Ende weist der Leuchtenarm 53 einen Hexapoden 58 mit einem Steuersignaleingang 59 auf, der mit dem Trägersystem 45 mechanisch verbunden ist.

Der Hexapod 58 weist sechs Beine auf, die jeweils ein erstes Bauteil mit einem gegenüberliegenden zweiten Bauteil des Leuchtearms 53 verbinden. Mehrere oder alle Beine sind motorisch längenverstellbar. Jedes Bein ist gelenkig an einem Befestigungspunkt am ersten Bauteil und an einem Befestigungspunkt am zweiten Bauteil befestigt. Die Befestigungspunkte sind so an den beiden Bauteilen angeordnet, dass die relative Anordnung und Ausrichtung der beiden Bauteile durch die Längen der Beine eindeutig bestimmt sind.

Die Position der Operationsleuchte 40, insbesondere des Reflektors 41 und der Kamera 43, und ihre Leuchtrichtung sind über motorische oder servomotorische Antriebe der Gelenke 54, 56, des Hexapoden 58 und im Deckenträger 51 verstellbar. Beispielsweise ermöglicht ein motorischer oder servomotorischer Antrieb im oder am Deckenträger 51 eine Rotation der Aufhängung 50 um eine vertikale Achse. Eine Verstellbarkeit um jeweils eine oder zwei Achsen an jedem der Gelenke 54, 56 - optional eine in Figur 1 nicht dargestellte teleskopische Verstellbarkeit des Leuchtenarms 53 oder weitere Gelenke - sowie der Hexapod 58 ermöglichen eine Verschiebung des Reflektors 41 und der Kamera 43 in allen Dimensionen und eine nahezu beliebige Ausrichtung des Reflektors 41 und der Kamera 43. Dabei ermöglichen die Rotierbarkeit um eine vertikale Achse am Deckenträger 51, die Gelenke 54, 56 und die optionale teleskopische Verstellbarkeit des Leuchtenarms 53 eine großräumige Verschiebung von Reflektor 41 und Kamera 43. Der Hexapod 58 ermöglicht in erster Linie ein Drehen und Schwenken um mehrere Achsen und ein kleinräumiges Verschieben des Reflektors 41 und der Kamera 43. Ferner können die Intensität, das Spektrum, die Größe des Lichtkegels bzw. des von ihm beleuchteten Bereichs und/oder die Gestalt des Lichtkegels über ein Steuersignal am Steuersignaleingang 42 gesteuert werden.

Obwohl der Deckenträger 51, die Gelenke 54, 56 und der Hexapod 58 mit jeweils eigenen Steuersignaleingängen 52, 55, 57, 59 dargestellt sind, kann alternativ an der gesamten Operationsleuchte 40 einschließlich der Aufhängung 50 ein einziger Steuersignaleingang vorgesehen sein. Dieser kann ferner mit dem Steuersignaleingang 42 der Lichtquelle und/oder dem Bildsignalausgang 44 der Kamera 43 integriert sein. Der Verlauf der Leitungen zwischen der Steuerung 60 einerseits und dem Bildsignalausgang 42 und den Steuersignaleingängen 33, 52, 55, 57, 59 andererseits kann von der schematischen Darstellung in Figur 1 abweichen. Insbesondere ist ein Verlauf innerhalb des Leuchtenarms 53 aus hygienischen und anderen praktischen Gründen vorteilhaft.

Eine Steuerung 60 ist mit der Allgemeinbeleuchtung 18 bzw. einer Leistungsversorgung oder einer Schalteinrichtung für die Allgemeinbeleuchtung 18, mit dem Steuersignaleingang 33 des Verstellantriebs 32 des Operationstischs 30, mit dem Steuersignaleingang 42 der Lichtquelle, mit dem Bildsignalausgang 44 der Kamera 43, mit dem Sensor 49, mit dem Steuersignaleingang 52 am Deckenträger 51, mit dem Steuersignaleingang 55 am ersten Gelenk 54, mit dem Steuersignaleingang 57 am zweiten Gelenk 56 und mit dem Steuersignaleingang 59 am Hexapoden 58 gekoppelt. Diese Kopplung kann wie in Figur 1 angedeutet über sternförmig zwischen der Steuerung 60 einerseits und den Steuersignaleingängen bzw. Bildsignalausgängen 33, 42, 44, 52, 55, 57, 59 andererseits verlaufenden Leitungen oder über einen oder mehrere Busse, beispielsweise einen SCB (Storz Communication Bus) oder einen CAN-Bus, erfolgen. Eine Kopplung über einen oder mehrere Bus-Systeme reduziert die Anzahl der erforderlichen Leitungen.

Die Kamera 43 kann zur Erfassung eines monochromatischen Bilds bzw. eines Schwarzweißbilds oder zur Erfassung von Bildern in mehreren verschiedenen Spektralbereichen bzw. zur Erfassung eines Farbbilds ausgebildet sein. Die spektrale Empfindlichkeit der Kamera kann auf den für das menschliche Auge sichtbare Spektralbereich beschränkt oder im Wesentlichen auf diesen Spektralbereich beschränkt sein. Alternativ oder zusätzlich ist die Kamera in anderen Spektralbereichen empfindlich, beispielsweise im nahen, mittleren und/oder fernen Infrarot.

Die Kamera 43 kann eine Stereokamera sein bzw. zwei Teilkameras umfassen, deren Aufnahmepositionen unterschiedlich sind. Aus den Unterschieden zweier von den Teilkameras gleichzeitig erfasster Bilder kann dreidimensionale Bildinformation gewonnen werden. Insbesondere könne Abstände von erfassten Gegenständen von der Stereokamera bestimmt werden. Alternativ ist die Kamera 43 beispielsweise eine Time-of-Flight-Kamera, die eine Bestimmung von dreidimensionaler Bildinformation aus Laufzeiten eines kurzen Lichtpulses ermöglicht.

Figur 2 zeigt eine schematische Darstellung einer Steuerung ähnlich der oben in Figur 1 dargestellten. Die in Figur 2 dargestellte Steuerung 60 unterscheidet sich von der oben anhand der Figur 1 dargestellten jedoch in der Anzahl der Ein- und Ausgänge. Die in Figur 2 dargestellte Steuerung 60 weist einen Bildsignaleingang 61, einen Positionierungssignaleingang 62 und einen Steuersignalausgang 63 auf. Der Bildsignaleingang 61 ist ausgebildet, um mit dem Bildsignalausgang 42 der Kamera 43 der Operationsleuchte 40 gekoppelt zu sein. Der Positionierungssignaleingang 62 ist ausgebildet, um parallel zum Steuersignaleingang 33 des Operationstischs 30 gekoppelt zu sein und wie dieser ein Positionierungssignal zur Steuerung des Operationstischs 30 zu empfangen. Alternativ oder zusätzlich ist der Positionierungseingang 62 ausgebildet, um ein Positionierungssignal zu empfangen, das eine Position oder eine Veränderung einer Position eines Gegenstands, mit dem die Operationsleuchte 40 kollidieren kann, darstellt. Der Steuersignalausgang 63 ist dazu ausgebildet, mit einem oder mehreren Steuersignaleingängen 42, 52, 55, 57, 59 der Operationsleuchte 40 gekoppelt zu sein.

Mit der unterbrochenen Linie 64 ist angedeutet, dass die Steuerung 60 alternativ nur einen oder zwei Signalein- und -ausgänge 61, 62 oder 63 aufweist, der die Funktionen des Bildsignaleingangs 61 und/oder des Positionierungssignaleingangs 62 und/oder des Steuersignalausgangs 63 integriert. Ein oder zwei solcher integrierter Signalein- und - ausgänge übernehmen die Aufgaben sowohl des Bildsignaleingangs 61 als auch des Positionierungssignaleingangs 62 und des Steuersignalausgangs 63. Diese ein oder zwei Signalein- und -ausgänge sind dann insbesondere jeweils für die Kopplung mit einem Bus (beispielsweise SCB oder CAN) ausgebildet, über den alle Steuersignale bzw. alle Steuersignale und alle Bildsignale übertragen werden. Nachfolgend wird jedoch wieder auf den Bildsignaleingang 61, den Positionierungssignaleingang 62 und den Steuersignalausgang 63 Bezug genommen. Die dabei beschriebene Funktionalität ist jedoch von Anzahl und Art der Ein- und Ausgänge unabhängig. Die Übertragung aller Signale über einen einzigen Bus oder wenige Bus-Systeme ist jedoch in vielen Fällen vorteilhaft.

Die Steuerung 60 umfasst eine erste Einrichtung 71, eine zweite Einrichtung 72, eine dritte Einrichtung 73, eine vierte Einrichtung 74, eine fünfte Einrichtung 75 und eine sechste Einrichtung 76. Die erste Einrichtung 71 ist mit dem Bildsignaleingang 61, der zweiten Einrichtung 72, der dritten Einrichtung 73, der fünften Einrichtung 75 und der sechsten Einrichtung 76 gekoppelt. Die zweite Einrichtung 72 ist mit der ersten Einrichtung 71 und der sechsten Einrichtung 76 gekoppelt. Die dritte Einrichtung 73 ist mit der ersten Einrichtung 71, der vierten Einrichtung 74 und der fünften Einrichtung 75 gekoppelt. Die vierte Einrichtung 74 ist mit der dritten Einrichtung 73 und der sechsten Einrichtung 76 gekoppelt. Die fünfte Einrichtung 75 ist mit der ersten Einrichtung 71, der dritten Einrichtung 73 und der sechsten Einrichtung 76 gekoppelt. Die sechste Einrichtung 76 ist mit dem Positionierungssignaleingang 62 und dem Steuersignalausgang 63 gekoppelt. Eine Kopplung beinhaltet dabei jeweils zumindest die Möglichkeit eines direkten oder indirekten Austauschs von Information bzw. eines Signals, das die Information darstellt.

Die erste Einrichtung 71 ist zur Bilderkennung, insbesondere zu Erkennung einer Hand eines Operateurs oder einer Markierung 24, 16 an einem Handschuh 22, einem Armband 23 oder eines Rands 15 eines Operationsfelds 14, ausgebildet. Die erste Einrichtung 71 ist ferner ausgebildet, um die Position der Hand oder der Markierung 16, 24 zu bestimmen. Die zweite Einrichtung 72 ist ausgebildet, um zu bestimmen, ob die von der ersten Einrichtung 71 bestimmte Position innerhalb eines vorbestimmten Bereichs liegt. Die dritte Einrichtung 73 ist ausgebildet, um eine Geschwindigkeit der Hand bzw. der Markierung 16, 24 zu ermitteln. Die vierte Einrichtung 74 ist ausgebildet, um zu bestimmen, ob die von der dritten Einrichtung 73 ermittelte Geschwindigkeit innerhalb eines vorbestimmten Bereichs liegt. Die fünfte Einrichtung 75 ist ausgebildet, um eine Geste der Hand des Operateurs oder der Markierung 24 zu erkennen. Die sechste Einrichtung 76 ist zur Erzeugung eines Steuersignals zur Steuerung der Operationsleuchte 40, optional zur Erzeugung eines Steuersignals für die Allgemeinbeleuchtung 18 und optional zur Erzeugung eines Steuersignals zur Steuerung des Operationstisches 30 ausgebildet.

Die erste Einrichtung 71 oder eine weitere, in Figur 2 nicht dargestellte Einrichtung kann ferner dazu ausgebildet sein, Sensorsignale von dem oder den Sensoren 49 zu empfangen und anhand dieser Sensorsignale die Identität der Markierung 24 und des Operateurs und/oder die Positionen der Markierungen 24, 16 zu bestimmen. Diese Sensoren 49 sind abgestimmt auf die Markierungen 24, 16 beispielsweise Ultraschallsensoren oder RFID-Sensoren.

Anstelle des Positionierungssignaleingangs 62 oder zusätzlich zu diesem kann ein Positionierungssignalausgang vorgesehen sein, über den die Steuerung 60 mit dem Steuersignaleingang 33 des Operationstischs 30 und/oder mit Steuersignaleingängen von einem oder mehreren Gegenständen, mit denen die Operationsleuchte 40 kollidieren kann, gekoppelt ist. In diesem Fall ist die sechste Einrichtung 76 auch zur Erzeugung eines Positionierungssignals für den Operationstisch 30 oder den Gegenstand ausgebildet.

Die erste Einrichtung 71 erhält über den Bildsignaleingang 61 ein Bildsignal und wertet dieses beispielsweise hinsichtlich der momentanen Anordnung einzelner vorbestimmter Bildelemente aus. Diese Bildelemente umfassen beispielsweise die Hand des Operateurs 20 oder Teile derselben oder eine oder mehrere Markierungen 16, 24. Diese Markierung unterscheidet sich und ist von anderen Gegenständen abgrenzbar beispielsweise aufgrund ihrer Farbe, ihres Reflexionsgrads, ihrer Form oder einer zeitlichen Modulation ihres Reflexionsgrads oder ihres Leuchtens. Die zweite Einrichtung 72, die dritte Einrichtung 73, die fünfte Einrichtung 75 und die sechste Einrichtung 76 erhalten die Position oder die Positionen und optional zusätzlich weitere Information über die Anordnung und Ausrichtung bzw. Orientierung des oder der vorbestimmten Merkmale in dem von der Kamera 43 erfassten Bild.

Alternativ oder zusätzlich erhält die erste Einrichtung 71 oder eine in Figur 2 nicht dargestellte Einrichtung von dem oder den Sensoren 49 (beispielsweise Ultraschallsensoren oder RFID-Sensoren) Sensorsignale. Die erste Einrichtung 71 oder eine in Figur 2 nicht dargestellte Einrichtung wertet diese Sensorsignal hinsichtlich der Identität bzw. Identitäten der Markierungen 24, 16 und/oder hinsichtlich der Positionen oder Orientierungen der Markierungen 24, 16 aus.

Die zweite Einrichtung 72 vergleicht die Position bzw. die Positionen mit einem vorbestimmten Bereich, beispielsweise mit dem Operationsfeld 14 und dessen Rand 15. Dieser Rand 15 des Operationsfelds kann durch eine oder mehrere Markierungen 16 markiert sein. Alternativ wird der Rand 15 des Operationsfelds 14 von der ersten Einrichtung 71 oder von der zweiten Einrichtung 72 beispielsweise mittels einer Bilderkennungs- oder Mustererkennungs-Software erkannt und lokalisiert.

Die dritte Einrichtung 73 ermittelt beispielsweise aus dem Vergleich von Positionen, die von der ersten Einrichtung 71 anhand mehrerer nacheinander erfasster Bilder bestimmt wurden, eine oder mehrere Geschwindigkeiten des bzw. der beobachteten Merkmale 16, 24. Die vierte Einrichtung 74 vergleicht die von der dritten Einrichtung 73 ermittelte Geschwindigkeit mit einem bzw. mehreren vorbestimmten Bereichen. Beispielsweise vergleicht die dritte Einrichtung 73 den Betrag und/oder eine oder mehrere kartesische Komponenten der Geschwindigkeit einzeln mit einer Obergrenze. Die fünfte Einrichtung 75 analysiert die von der ersten Einrichtung 71 bestimmten Positionen und die von der dritten Einrichtung 73 bestimmten Geschwindigkeiten und identifiziert eine oder mehrere Gesten der Hand des Operateurs 20.

Die sechste Einrichtung 76 empfängt von der ersten Einrichtung 71 die Position oder Positionen und optional die Ausrichtung relevanter Merkmale in dem von der Kamera 43 erfassten Bild. Alternativ oder zusätzlich empfängt die sechste Einrichtung 76 von der zweiten Einrichtung 72 Information darüber, ob die von der ersten Einrichtung 71 bestimmte Position oder bestimmten Positionen innerhalb eines bzw. mehrerer vorbestimmter Bereiche liegen. Alternativ oder zusätzlich empfängt die sechste Einrichtung 76 von der vierten Einrichtung 74 Information darüber, ob die von der dritten Einrichtung 73 bestimmte Geschwindigkeit oder bestimmten Geschwindigkeiten innerhalb eines bzw. mehrerer vorbestimmter Bereiche liegen. Alternativ oder zusätzlich empfängt die sechste Einrichtung 76 von der fünften Einrichtung 75 Information zu von der Hand des Operateurs 20 ausgeführten Gesten. Alternativ oder zusätzlich empfängt die sechste Einrichtung 76 ein Positionierungssignal, das eine Position oder eine Veränderung einer Position des Operationstischs 30 oder eines Gegenstands, mit dem die Operationsleuchte 40 kollidieren kann, darstellt.

Aufgrund dieser Informationen erzeugt die sechste Einrichtung 76 ein oder mehrere Steuersignale für die Antriebe in bzw. an dem Deckenträger 51, der Gelenke 54, 56 und des Hexapoden 58. Die sechste Einrichtung 76 erzeugt das bzw. die Steuersignale so, dass das von der Operationsleuchte 40 erzeugte Licht bzw. der Lichtkegel auch bei einer Bewegung des Operationstischs 30 und des Patienten 13 im Operationssaal 10 auf das Operationsfeld 14 gerichtet ist und dieses vollständig und gleichmäßig ausleuchtet. Alternativ oder zusätzlich erzeugt die sechste Einrichtung 76 das oder die Steuersignale so, dass das von der Operationsleuchte 40 erzeugte Licht immer die Hand des Operateurs 20 oder eine Markierung 24 an der Hand des Operateurs 20 und deren Umgebung ausleuchtet. Alternativ oder zusätzlich erzeugt die sechste Einrichtung 76 das oder die Steuersignale so, dass der Operateur 20 mit vorbestimmten Gesten, die er mit einer oder beiden Händen ausführt, die Richtung, eine spektrale Eigenschaft des von der Operationsleuchte 40 erzeugten Lichts oder eine Gestalt des von dem Licht beleuchteten Bereichs beeinflussen kann. Alternativ oder zusätzlich erzeugt die sechste Einrichtung 76 das bzw. Steuersignale so, dass Bewegungen oder Gesten der Hand des Operateurs 20 nicht bei der Steuerung der Operationsleuchte 40 berücksichtigt werden, wenn sie außerhalb eines vorbestimmten Bereichs, beispielsweise außerhalb des Operationsfelds 14, oder mit einer zu hohen Geschwindigkeit ausgeführt werden.

Alternativ oder zusätzlich erzeugt die sechste Einrichtung 76 ein Steuersignal für den Steuersignaleingang 42, über den beispielsweise die Intensität, spektrale Eigenschaften, Größe oder Form des Lichtkegels bzw. des von der Operationsleuchte 40 beleuchteten Bereichs gesteuert werden.

Alternativ ist die sechste Einrichtung 76 ausgebildet, um ein Positionierungssignal für den Operationstisch 30 oder für einen Gegenstand, mit dem die Operationsleuchte kollidieren kann, zu erzeugen. Unabhängig davon, ob die Steuerung ausgebildet ist, um ein Positionierungssignal zu empfangen oder zu erzeugen und zu senden, ist die sechste Einrichtung 76 ausgebildet, um das Steuersignal (und gegebenenfalls das Positionierungssignal) so zu erzeugen, dass die Operationsleuchte gegebenenfalls mit dem Gegenstand nicht kollidiert.

Die erste Einrichtung 71 oder eine weitere, in Figur 2 nicht dargestellte Einrichtung der Steuerung 60 kann ausgebildet sein, um zusätzlich zur Erkennung einer Hand eines Operateurs 20 einen Arm 21, einen Kopf 25 oder einen oder mehrere andere Körperteile von medizinischem Personal zu erkennen und die Positionen dieser Körperteile oder die Positionen von medizinischem Personal zu bestimmen. In diesem Fall können die Steuerung 60 und die sie bildenden Einrichtungen 71, 72, 73, 74, 75, 76 so ausgebildet sein, dass das Steuersignal in Abhängigkeit von den Positionen des medizinischen Personals oder von Armen 21, Köpfen 25 oder anderen Körperteilen des medizinischen Personals erzeugt wird. Insbesondere ist die Steuerung 60 ausgebildet, um das Steuersignal so zu erzeugen, dass eine Abschattung des von der Operationsleuchte 40 erzeugte Licht durch Arme, Köpfe oder Oberkörper von medizinischem Personal verhindert wird.

Ferner kann die Steuerung 60 ausgebildet sein, um ein Bildsignal einer auf den Kopf 25 des Operateurs 20 oder anderen medizinischen Personals gerichteten Kamera zu empfangen, und um in dem Bildsignal den Kopf 25 des Operateurs 20 oder anderen medizinischen Personals und eine Bewegung des Kopfs 25 zu erkennen und die Operationsleuchte 40 in Abhängigkeit von einer als Steuerbefehl erkannten Bewegung des Kopfs 25 zu steuern. Alternativ oder zusätzlich kann die Steuerung 60 ausgebildet sein, um ein Auge 27 oder beide Augen des medizinischen Personals 20, eine Blickrichtung, eine Änderung der Blickrichtung, ein Blinzeln und/oder eine andere Augenbewegung zu erkennen und die Operationsleuchte 40 in Abhängigkeit von einer als Steuerbefehl erkannten Augenbewegung zu steuern. Alternativ oder zusätzlich kann die Steuerung 60 ausgebildet sein, um eine Mimik des Gesichts 26 des Operateurs zu erkennen und die Operationsleuchte 40 in Abhängigkeit von einer als Steuerbefehl erkannten Mimik zu steuern.

Ferner kann die Steuerung 60 ausgebildet sein, um die Operationsleuchte 40 so zu steuern, dass eine Blendung von medizinischem Personal direkt durch die Operationsleuchte 40 oder durch Reflexionen an medizinischem Gerät vermieden wird.

Wenn die Operationsleuchte mehrere Einzelleuchten umfasst, kann die Steuerung 60 ausgebildet sein, um Einzelleuchten, deren Licht abgeschattet würde, auszuschalten oder deren Leistung zu reduzieren, und gleichzeitig zur Kompensation andere Einzelleuchten zuzuschalten. Ferner kann die Steuerung 60 ausgebildet sein, um die Richtung und/oder die Fokussierung bzw. die Divergenz des von Einzelleuchten bereitgestellten Lichts durch Verstellen von Reflektoren der Einzelleuchten einzustellen.

Die Einrichtungen 71, 72, 73, 74, 75, 76 der Steuerung 60 können separate Einrichtungen sein, wie es in Figur 2 dargestellt ist. Alternativ werden die Funktionalitäten von mehreren Einrichtungen 71, 72, 73, 74, 75, 76 in einer Einrichtung integriert. Ferner können die Funktionalitäten von allen oder einigen der Einrichtungen 71, 72, 73, 74, 75, 76 von Computerprogrammen, Funktionen, Teilprogrammen oder Threads realisiert sein, die in einem oder mehreren Prozessoren ablaufen.

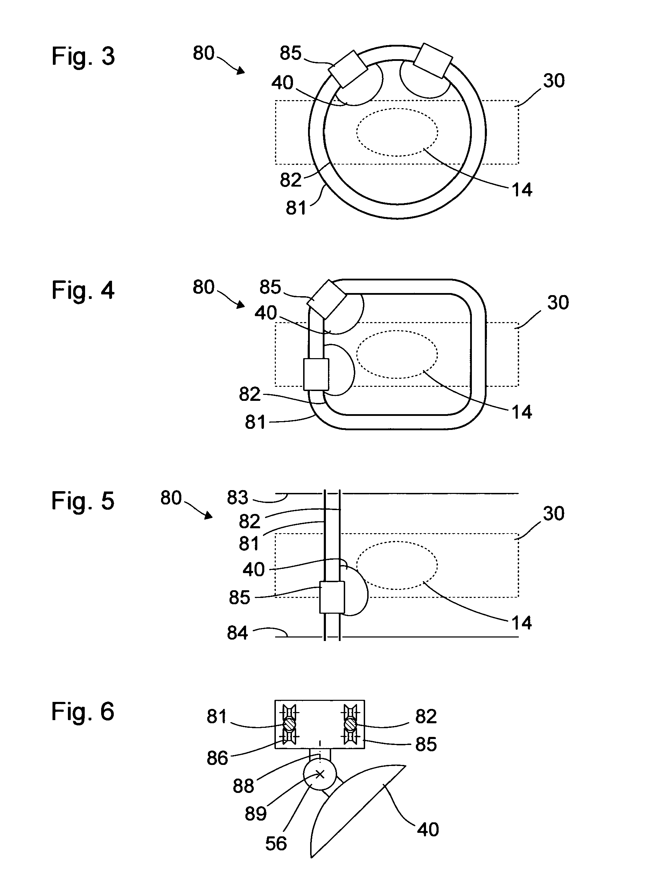

Die Figuren 3 bis 5 zeigen schematische Darstellungen dreier verschiedener Ausführungsformen einer Schieneneinrichtung 80 mit jeweils einer oder mehreren Operationsleuchten 40. Die dargestellten Schieneneinrichtungen sind Alternativen zu der oben anhand der Figur 1 dargestellten Aufhängung einer Operationsleuchte an einem Arm mit mehreren Gelenken. Ferner können die in Figur 1 dargestellte Aufhängung und die in den Figuren 3 bis 5 dargestellten Schieneneinrichtungen sich ergänzen bzw. kombiniert werden. In den Figuren 3 bis 5 ist jeweils eine Draufsicht dargestellt, wobei die Zeichenebene parallel zum Boden des Operationssaals ist. In unterbrochener Linie sind jeweils der Operationstisch 30 und das auszuleuchtende Operationsfeld 14 angedeutet.

Bei der in Figur 3 dargestellten Ausführungsform umfasst die Schieneneinrichtung 80 eine erste Schiene 81 und eine zweite Schiene 82, die parallel zueinander angeordnet sind. Sowohl die erste Schiene 81 als auch die zweite Schiene 82 ist kreisförmig. Beide Schienen 81, 82 sind in einer Ebene und konzentrisch angeordnet. Eine, zwei oder mehr Laufkatzeneinrichtungen 85 sind entlang den Schienen 81, 82 verfahrbar, insbesondere elektromotorisch verfahrbar. Die Laufkatzeneinrichtungen 85 können jeweils beide Schienen 81, 82 umgreifen, um auch bei Einwirkung einer Kraft oder eines Moments auf die Laufkatzeneinrichtung 85 ein Abkippen und Abstürzen von den Schienen 81, 82 zu verhindern.

An jeder Laufkatzeneinrichtung 85 ist eine Operationsleuchte 40 angeordnet. Insbesondere ist die Operationsleuchte 40 jeweils unter der Laufkatzeneinrichtung 85 angeordnet. Ein Ausführungsbeispiel einer Laufkatzeneinrichtung 85 ist unten mit Bezug auf Figur 6 näher beschrieben.

Die beiden Laufkatzeneinrichtungen 85 mit den daran angeordneten Operationsleuchten 40 sind insbesondere unabhängig voneinander entlang den Schienen verfahrbar. Zur Vermeidung einer Kollision der Laufkatzeneinrichtungen 85 oder der Operationsleuchten 40 kann die oben anhand der Figuren 1 und 2 dargestellte Steuerung 60 eine Kollisionsvermeidungseinrichtung aufweisen. Alternativ ist ein Kollisionsvermeidungsalgorithmus in einer Software der Steuerung 60 enthalten. Ferner kann eine Kollision zwischen den Laufkatzeneinrichtungen 85 oder den Operationsleuchten 40 mittels Annäherungssensoren oder anderer Einrichtungen an den Laufkatzeneinrichtungen 85 oder an den Operationsleuchten 40 vermieden werden. Alternativ werden Folgen einer Kollision mittels stoßabsorbierenden Elementen vermieden oder vermindert.

Ein Vorteil der in Figur 3 gezeigten kreisförmigen Gestalt der Schienen 81,82 ist, dass die Operationsleuchte 40 oder die Operationsleuchten 40 um das Operationsfeld 14 herum gefahren werden können, um das Operationsfeld 14 aus einer beliebigen Richtung zu beleuchten. Anstelle einer Kreisform können die Schienen 81, 82 eine beliebige andere Gestalt aufweisen, beispielsweise eine elliptische, U-förmige, gerade oder S-förmige Gestalt. Eine elliptische Gestalt der Schienen 81, 82 kann der kreisförmigen Gestalt gegenüber den Vorteil aufweisen, dass die elliptische Gestalt an die typischerweise längliche Ausdehnung des auf dem Operationstisch 30 liegenden Körpers des Patienten angepasst sein kann.

Figur 4 zeigt eine schematische Darstellung eines Beispiels, bei dem die Schienen eine im Wesentlichen rechteckige Gestalt mit abgerundeten Ecken aufweisen. Diese Gestalt der Schienen 81, 82 erfordert - ähnlich wie beispielsweise eine elliptische Gestalt oder eine S-förmige Gestalt der Schienen - eine Ausbildung der Laufkatzeneinrichtung 85, die eine Anpassung an eine variierende Krümmung der Schienen 81, 82 ermöglicht. Ein Vorteil der in Figur 4 gezeigten rechteckigen Gestalt der Schienen 81, 82 kann in geringeren Fertigungskosten für gerade und lediglich einen einzigen Krümmungsradius aufweisende kreisbogenförmige Schienen 81, 82 bestehen.

Figur 5 zeigt eine schematische Darstellung einer weiteren Ausführungsform einer Schieneneinrichtung 80. Eine Laufkatzeneinrichtung 85 mit einer Operationsleuchte 40 ist an einer ersten Schien 81 und einer zweiten Schiene 82 angeordnet. Die erste Schiene 81, und die zweite Schiene 82 sind jeweils gerade und parallel zu einander angeordnet. Enden der ersten Schiene 81 und der zweiten Schiene 82 sind entlang einer dritten Schiene 83 und einer vierten Schiene 84 verfahrbar. Die dritte Schiene 83 und die vierte Schiene 84 sind jeweils gerade und parallel zu einander angeordnet. Die erste Schiene 81 und die zweite Schiene 82 sind im Wesentlichen senkrecht zu der dritten Schiene 83 und der vierten Schiene 84 angeordnet.

Die Laufkatzeneinrichtung 85 und mit ihr die Operationsleuchte 40 sind entlang der ersten Schiene 81 und der zweiten Schiene 82 verfahrbar, insbesondere elektromotorisch verfahrbar. Unabhängig davon sind die erste Schiene 81 und die zweite Schiene 82 entlang der dritten Schiene 83 und der vierten Schiene 84 verfahrbar, insbesondere elektromotorisch verfahrbar. Dadurch kann die Operationsleuchte innerhalb einer rechteckigen Fläche, deren Abmessungen im Wesentlichen durch die Längen der Schienen 81, 82, 83, 84 gegeben ist, beliebig positioniert werden. Dies ermöglicht eine für viele Anwendungsfälle optimale Anordnung der Operationsleuchte.

Abweichend von der Darstellung in Figur 5 können mehrere Operationsleuchten 40 an jeweils einer Laufkatzeneinrichtung 85 vorgesehen sein, die unabhängig voneinander oder weitgehend unabhängig voneinander positionierbar sind. Insbesondere ist für jede Laufkatzeneinrichtung 85 ein eigenes Paar Schienen 81, 82 vorgesehen, das entlang der dritten Schiene 83 und der vierten Schiene 84 verfahrbar ist.

Figur 6 zeigt eine schematische Darstellung einer Laufkatzeneinrichtung 85 an einer ersten Schiene 81 und einer zweiten Schiene 82, wie sie beispielsweise oben anhand der Figuren 3 bis 5 dargestellt sind. Die Zeichenebene der Figur 6 ist senkrecht zu den Zeichenebenen der Figuren 3 bis 5. Bei dem in Figur 6 gezeigten Beispiel weisen die Schienen 81, 82 jeweils einen kreisförmigen oder im Wesentlichen kreisförmigen Querschnitt auf. Die Laufkatzeneinrichtung 85 ist mittels mehrerer Laufräder 86 an den Schienen 81, 82 geführt und entlang diesen verfahrbar.

Die Laufkatzeneinrichtung 85 umgreift die Schienen 81, 82 vollständig. Sowohl über als auch unter den Schienen 81, 82 sind Laufräder 86 angeordnet. Dadurch kann die Laufkatzeneinrichtung 85 auch bei Einwirkung von Kräften oder Momenten nicht von den Schienen 81, 82 abgehoben werden oder abstürzen. Die Laufkatzeneinrichtung 85 kann eine Antriebseinrichtung aufweisen, die in Figur 6 nicht dargestellt ist. Insbesondere kann die Laufkatzeneinrichtung 85 einen oder mehrere auf Laufräder 86 wirkende Elektromotoren oder einen Linearantrieb aufweisen.

Die Laufkatzeneinrichtung 85 ist über ein Gelenk 56 mit einer Operationsleuchte 40 gelenkig verbunden. Die Operationsleuchte ist um eine erste Schwenkachse 88, die parallel zur Zeichenebene der Figur 6 ist, und um eine zweite Schwenkachse 89 schwenkbar. Die zweite Schwenkachse 89 ist senkrecht zur ersten Schwenkachse 88, rotiert mit der Operationsleuchte 40 um die erste Schwenkachse 88 und ist bei der in Figur 6 gezeigten Position der Operationsleuchte 40 senkrecht zur Zeichenebene der Figur 6.

Die Verfahrbarkeit der Laufkatzeneinrichtung 85 mit der Operationsleuchte 40 entlang der ersten Schiene 81 und der zweiten Schiene 82, gegebenenfalls die Verfahrbarkeit der ersten Schiene 81 und der zweiten Schiene 82 entlang der dritten Schiene 83 und der vierten Schiene 84 und die Schwenkbarkeit der Operationsleuchte um die erste Schwenkachse 88 und die zweite Schwenkachse 89 ermöglicht eine weitgehend beliebige Positionierung und Ausrichtung der Operationsleuchte 40. Dadurch kann die Operationsleuchte 40 oder können mehrere Operationsleuchten 40 in vielen oder allen Anwendungsfällen so angeordnet und ausgerichtet werden, dass das Operationsfeld 14 optimal ausgeleuchtet ist.

Bei allen oben anhand der Figuren3 bis 6 dargestellten Beispielen und Varianten können anstelle zweier paralleler Schienen nur eine Schiene oder drei oder mehr parallele Schienen vorgesehen sein. Die Querschnitte der Schiene bzw. der Schienen sind beispielsweise kreisförmig, elliptisch, rechteckig, T-förmig, L-förmig oder I-förmig.

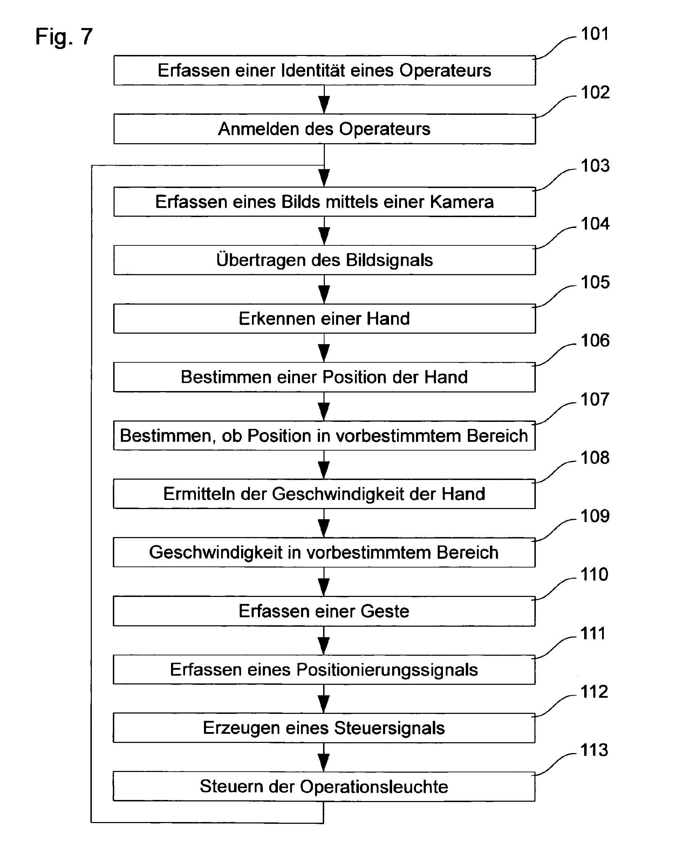

Figur 7 zeigt ein schematisches Flussdiagramm eines Verfahrens zum Betreiben einer Operationsleuchte zum Erzeugen von Licht. Obwohl das Verfahren auch mit Vorrichtungen ausführbar ist, die sich von den oben anhand der Figuren 1 und 2 dargestellten unterscheiden, werden nachfolgend beispielhaft Bezugszeichen aus den Figuren 1 und 2 verwendet, um das Verständnis zu erleichtern.

Bei einem ersten Schritt 101 wird eine Identität des Operateurs 20 erfasst. Dies erfolgt beispielsweise anhand einer Form, einer Farbe, eines Reflexionsgrads oder einer zeitlichen Modulation einer optisch (im infraroten, sichtbaren oder ultravioletten Spektralbereich) erfassbaren Markierung an einem Handschuh 22 oder einem Armband 23 des Operateurs 20 durch eine Kamera 43 in der Operationsleuchte 40. Alternativ oder zusätzlich erfolgt die Identifizierung über einen Transponder (beispielsweise einen RFID-Transponder) am oder im Handschuh 22 oder Armband 23 des Operateurs 20 oder auf andere Weise. Bei einem zweiten Schritt 102 wird der Operateur 20 in einem Operationssystem angemeldet, beispielsweise um einen entsprechenden Eintrag in einem Protokoll zu erzeugen, um von dem Operateur 20 bevorzugte Einstellungen an Geräten im Operationssaal 10 vorzunehmen oder um Parameter zu laden, die zur Erkennung von Gesten des Operateurs verwendet werden.

Bei einem dritten Schritt 103 wird mittels der Kamera 43 der Operationsleuchte 40 ein Bild des Operationsfelds 14 und der Hand des Operateurs 20 mit dem Handschuh 22 und/oder dem Armband 23 und einer Markierung 24 daran erfasst. Die Kamera 43 erzeugt ein Bildsignal, das bei einem vierten Schritt 104 von der Kamera 43 zu einer Steuerung 60 übertragen wird. Das Bildsignal ist beispielsweise ein analoges oder digitales Signal. Bei einer Übertragung über einen Bus (beispielsweise SCB oder CAN) erfolgt die Übertragung des Bildsignals beispielsweise in Paketen.

Bei einem fünften Schritt 105 wird die Hand des Operateurs 20 beispielsweise aufgrund ihrer Farbe oder Form oder aufgrund der Markierung 24 am Armband 23 oder am Handschuh 22 des Operateurs 20 erkannt. Dazu wird beispielsweise eine Bilderkennungssoftware verwendet. Bei einem sechsten Schritt 106, der mit dem fünften Schritt 105 integriert sein kann, wird eine Position der Hand bestimmt. Diese Position ist beispielsweise eine Position relativ zum von der Kamera 43 erfassten Bild bzw. seinen Rändern. Alternativ ist die Position eine Position relativ zum Operationsfeld 14 bzw. seinen Rändern 15 oder eine Position in einem festen Koordinatensystem im Operationssaal 10.

Bei der Bestimmung der Position der Hand im sechsten Schritt 106 können alternativ oder zusätzlich zu den Bildsignalen der Kamera 43 Sensorsignale von einem oder mehreren weiteren Sensoren 49 verwendet werden. Diese Sensoren 49 sind an die Markierung 24 angepasst, beispielsweise Ultraschallsensoren oder RFID-Sensoren.

Bei einem siebten Schritt 107 wird bestimmt, ob die beim sechsten Schritt 106 bestimmte Position in einem vorbestimmten Positions-Bereich liegt. Bei einem achten Schritt 108 wird eine Geschwindigkeit der Hand des Operateurs 20 bestimmt, beispielsweise durch einen Vergleich der Positionen der Hand, die aufgrund mehrerer Bilder zu verschiedenen Zeitpunkten bestimmt wurden. Bei einem neunten Schritt 109 wird bestimmt, ob die beim achten Schritt 108 ermittelte Geschwindigkeit in einem vorbestimmten GeschwindigkeitsBereich liegt.

Bei einem zehnten Schritt 110 wird eine von der Hand des Operateurs 20 ausgeführte Geste erfasst, beispielsweise durch Vergleich der Positionen und Geschwindigkeiten der Hand, die zu verschiedenen Zeitpunkten bestimmt bzw. ermittelt wurden. Gesten sind beispielsweise linear oszillierende oder kreisende Bewegungen mit bestimmter Richtung oder Rotationsrichtung, das Bilden einer Faust, das Abspreizen von einem oder mehreren Fingern oder vorbestimmte Bewegungen beider Hände relativ zu einander.

Bei einem elften Schritt 111 werden ein oder mehrere Positionierungssignale erfasst, welche die Soll- oder Ist-Position oder eine Veränderung der Position des Operationstischs 30, einer weiteren Operationsleuchte oder eines anderen Gegenstands, mit dem die Operationsleuchte 40 kollidieren kann, darstellt.

Bei einem zwölften Schritt 112 werden ein oder mehrere Steuersignale zum Steuern der Richtung, Intensität oder spektralen Eigenschaft des Lichts der Operationsleuchte 40 oder einer Gestalt des von dem Licht beleuchteten Bereichs erzeugt. Das oder die Steuersignale werden in Abhängigkeit von der oder den beim sechsten Schritt 106 bestimmten Positionen, von der beim zehnten Schritt 110 erfassten Geste und von dem oder den beim elften Schritt 111 erfassten Positionierungssignalen sowie abhängig davon, ob die Position oder Positionen und die Geschwindigkeit oder Geschwindigkeiten innerhalb der vorbestimmten Bereiche liegen, erzeugt. Bei einem dreizehnten Schritt 113 wird die Operationsleuchte 40 mittels des beim zwölften Schritt 112 erzeugten Steuersignals gesteuert.

Der dritte Schritt 103, der vierte Schritt 104, der fünfte Schritt 105, der sechste Schritt 106, der siebte Schritt 107, der achte Schritt 108, der neunte Schritt 109, der zehnte Schritt 110, der elfte Schritt 111, der zwölft Schritt 112 und der dreizehnte Schritt 113können periodisch oder nicht-periodisch wiederholt und teilweise in einer von der Darstellung in Figur 7 abweichenden Reihenfolge oder teilweise gleichzeitig ausgeführte werden. Das Verfahren kann durch weitere, in Figur 7 nicht dargestellte Figuren ergänzt oder durch Verzicht auf einzelne der in Figur 7 dargestellten Schritte modifiziert werden.

### Bezugszeichen

- 10: Operationssaal 10
- 11: Boden des Operationssaals 10
- 12: Decke des Operationssaals 10
- 13: Patient
- 14: Operationsfeld
- 15: Rand des Operationsfelds 14
- 16: Markierung am Rand 15 des Operationsfeld 14
- 18: Allgemeinbeleuchtung des Operationssaals 10
- 20: Operateur
- 21: Arm des Operateurs 20 mit Hand
- 22: Handschuh des Operateurs 20
- 23: Armband am Arm 21 des Operateurs 20
- 24: Markierung am Armband 23
- 25: Kopf des Operateurs 20
- 26: Gesicht des Operateurs 20
- 27: Auge des Operateurs 20
- 30: Operationstisch
- 31: Liegefläche des Operationstischs 30
- 32: Verstellantriebe des Operationstischs 30
- 33: Steuersignaleingang des Verstellantriebs 32
- 40: Operationsleuchte
- 41: Reflektor der Operationsleuchte 40
- 42: Bildsignalausgang der Operationsleuchte 40
- 43: Kamera in der Operationsleuchte 40
- 44: Bildsignalausgang der Operationsleuchte
- 45: Trägersystem
- 49: Sensor
- 50: Aufhängung der Operationsleuchte 40
- 51: Deckenträger
- 52: Steuersignaleingang am Deckenträger 51
- 53: Leuchtenarm
- 54: erstes Gelenk am Leuchtenarm 53
- 55: Steuersignaleingang am ersten Gelenk 54
- 56: zweites Gelenk am Leuchtenarm 53
- 57: Steuersignaleingang am zweiten Gelenk 56
- 58: Hexapod
- 59: Steuersignaleingang am Hexapoden 58
- 60: Steuerung
- 61: Bildsignaleingang der Steuerung 60
- 62: Positionierungssignaleingang der Steuerung 60 für Operationstisch 30
- 63: Steuersignalausgang der Steuerung 60 für Operationstisch 30
- 64: interne Verbindungsleitung
- 71: erste Einrichtung (Bilderkennung)
- 72: zweite Einrichtung (Position im Operationsfeld?)
- 73: dritte Einrichtung (Ermittlung der Geschwindigkeit)
- 74: vierte Einrichtung (Geschwindigkeit innerhalb vorbestimmten Bereichs?)
- 75: fünfte Einrichtung (Gestenerkennung)
- 76: sechste Einrichtung (Steuerung der Operationsleuchte 40)
- 80: Schieneneinrichtung
- 81: erste Schiene
- 82: zweite Schiene
- 83: dritte Schiene
- 84: vierte Schiene
- 85: Laufkatzeneinrichtung
- 86: Laufrad
- 88: erste Schwenkachse
- 89: zweite Schwenkachse

- 101: erster Schritt
- 102: zweiter Schritt
- 103: dritter Schritt
- 104: vierter Schritt
- 105: fünfter Schritt
- 106: sechster Schritt
- 107: siebter Schritt
- 108: achter Schritt
- 109: neunter Schritt
- 110: zehnter Schritt
- 111: elfter Schritt
- 112: zwölfter Schritt
- 113: dreizehnter Schritt

## Patentansprüche

1. Steuerung (60) zum Steuern einer Operationsleuchte (40) zum Erzeugen von Licht, mit:
einem Bildsignaleingang (61) zum Empfangen eines Bildsignals von einer Kamera (43) zum Erfassen eines Bilds eines Operationsfelds (14);
einem Steuersignalausgang (63) zum Ausgeben eines Steuersignals zur Steuerung zumindest entweder einer Richtung oder einer spektrale Eigenschaft des Lichts oder
einer Größe oder Gestalt des von dem Licht beleuchteten Bereichs an die Operationsleuchte (40);
**dadurch gekennzeichnet, dass**
die Steuerung (60) ausgebildet ist, um anhand des Bildsignals eine Geste einer Hand, oder eines Unterarms (21), des medizinischen Personals (20) zu erkennen und das Steuersignal in Abhängigkeit von der Geste zu erzeugen.

2. Steuerung (60) nach dem vorangehenden Anspruch, wobei die Steuerung (60)ferner ausgebildet ist, um anhand des Bildsignals zu bestimmen, ob eine Position eines Handbereichs (21, 22, 23, 24) von medizinischem Personal (20) in einem vorbestimmten Raumbereich (14) liegt, und um das Steuersignal ferner in Abhängigkeit davon zu erzeugen, ob die Position des Handbereichs (21, 22, 23, 24) in dem vorbestimmten Raumbereich liegt.

3. Steuerung (60) nach einem der vorangehenden Ansprüche, wobei die Steuerung (60) ferner dazu ausgebildet ist, um eine Geschwindigkeit eines Handbereichs (21, 22, 23, 24) von medizinischem Personal (20) zu bestimmen, um zu bestimmen, ob die Geschwindigkeit des Handbereichs (21, 22, 23, 24) des medizinischen Personals (20) in einem vorbestimmten Geschwindigkeitsbereich liegt, und um das Steuersignal ferner in Abhängigkeit davon zu erzeugen, ob die Geschwindigkeit des Handbereichs (21, 22, 23, 24) des medizinischen Personals (20) in dem vorbestimmten Geschwindigkeitsbereich liegt.

4. Steuerung (60) nach einem der vorangehenden Ansprüche, wobei die Steuerung (60) ferner ausgebildet ist, um ein Positionierungssignal zu empfangen, das eine Position oder eine Veränderung der Position eines verstellbaren Operationstischs (30) oder eines Gegenstands, mit dem die Operationsleuchte (40) kollidieren kann,
darstellt, und um das Steuersignal in Abhängigkeit von dem Positionierungssignal zu erzeugen.

5. Steuerung (60) nach einem der vorangehenden Ansprüche, wobei die Steuerung (60) ferner ausgebildet ist, um zumindest entweder eine Bewegung eines Kopfs, eine Mimik eines Gesichts oder eine Augenbewegung von medizinischem Personal (20) zu erkennen und das Steuersignal in Abhängigkeit von der Bewegung des Kopfs, der Mimik des Gesichts oder der Augenbewegung zu erzeugen.

6. Operationsleuchtensystem mit einer Steuerung nach einem der vorangehende Ansprüche und einer Operationsleuchte (40) zum Erzeugen von Licht, wobei die Operationsleuchte umfasst:
einer Kamera (43) zum Erfassen eines Bilds eines Operationsfelds (14) und zum Erzeugen eines Bildsignals;
einem Bildsignalausgang (44) zum Ausgeben des Bildsignals an eine Steuerung (60);
einem Steuersignaleingang (42, 52, 55, 57, 59) zum Empfangen eines Steuersignals von der Steuerung (60);
wobei die Operationsleuchte (40) ausgebildet ist, um zumindest entweder eine Richtung oder eine spektrale Eigenschaft des Lichts oder eine Größe oder Gestalt des von dem Licht beleuchteten Bereichs in Abhängigkeit von dem Steuersignal zu verändern.

7. Operationsleuchtensystem nach dem vorangehenden Anspruch, bei der die Operationsleuchte (40) zumindest entweder an einer Schieneneinrichtung angeordnet ist oder eine Mehrzahl von Einzelleuchten umfasst, die in einem Array oder auf andere gleichförmige oder ungleichförmige Art angeordnet sind.

8. Verfahren zum Betreiben einer Operationsleuchte (40) zum Erzeugen von Licht, mit folgenden Schritten:
Erfassen (103) eines Bilds des Operationsfelds (14) und Erzeugen eines Bildsignals mittels einer Kamera (43);
Übertragen (104) des Bildsignals von der Kamera (43) zu einer Steuerung (60);
**gekennzeichnet durch** die folgenden Schritte:
Erkennen einer Hand oder eines Unterarmsvon medizinischem Personal (20) in dem Bildsignal;
Bestimmen (106) einer Geste der Hand, oder des Unterarms (21), des medizinischen Personals anhand des Bildsignals,
Erzeugen (113) eines Steuersignals in Abhängigkeit von dem Bildsignal und in Abhängigkeit von der bestimmten Geste;
Steuern (114) zumindest entweder einer Richtung oder einer spektralen Eigenschaft des Lichts oder einer Größe oder Gestalt des von dem Licht beleuchteten Bereichs mittels des Steuersignals.

9. Verfahren nach dem vorangehenden Anspruch, ferner mit folgendem Schritt:
Bestimmen (107), ob die Position des Handbereichs (21, 22, 23, 24) des medizinischen Personals (20) in einem vorbestimmten Raumbereich (14) liegt,
wobei das Steuersignal ferner in Abhängigkeit davon erzeugt wird, ob die Position des Handbereichs (21, 22, 23, 24) in dem vorbestimmten Raumbereich liegt.

10. Verfahren nach einem der Ansprüche 8 und 9, ferner mit folgendem Schritt:
Bestimmen (108) der Geschwindigkeit eines Handbereichs (21, 22, 23, 24) des medizinischen Personals (20);
Bestimmen (109), ob die Geschwindigkeit des Handbereichs (21, 22, 23, 24) des medizinischen Personals (20) in einem vorbestimmten Geschwindigkeitsbereich (14) liegt,
wobei das Steuersignal ferner in Abhängigkeit davon erzeugt wird, ob die Geschwindigkeit des Handbereichs (21, 22, 23, 24) des medizinischen Personals (20) in dem vorbestimmten Geschwindigkeitsbereich (14) liegt.

11. Verfahren nach einem der Ansprüche 8 bis 10, ferner mit folgendem Schritt:
Erfassen (111) eines Positionierungssignals, das eine Position oder eine Veränderung der Position eines Operationstischs (30) oder eines Gegenstands, mit dem die Operationsleuchte (40) kollidieren kann, anzeigt,
wobei das Steuersignal in Abhängigkeit von dem Positionierungssignal erzeugt wird.

12. Verfahren nach einem der Ansprüche 8 bis 11, ferner mit folgendem Schritt:
Erfassen (101) einer Identität des medizinischen Personals (20) mittels einer Kamera (43) oder eines Sensors (49).

13. Verfahren nach dem vorangehenden Anspruch, ferner mit folgendem Schritt:
Anmelden (102) des medizinischen Personals (20) in einem Operationssystem.

## Claims

1. Control unit (60) for controlling an operation light (40) for generating light, comprising:
an image signal input (61) for receiving an image signal from a camera (43) for acquiring an image of an operation field (14);
a control signal output (63) for transmitting a control signal for controlling at least a direction or a spectral property of the light, or a dimension or form of the region illuminated by the light, to the operation light (40);
**characterized in that**
the control unit (60) is embodied to identify a gesture of a hand or of a forearm (21) of the medical staff (20) on the basis of the image signal and to generate the control signal depending on the gesture.

2. Control unit (60) according to the preceding claim, wherein the control unit (60) is furthermore embodied to determine whether a position of a hand region (21, 22, 23, 24) of medical staff (20) lies in a predetermined spatial region (14) on the basis of the image signal and furthermore to generate the control signal depending on whether the position of the hand region (21, 22, 23, 24) lies in the predetermined spatial region.

3. Control unit (60) according to one of the preceding claims, wherein the control unit (60) is furthermore embodied to determine a speed of a hand region (21, 22, 23, 24) of medical staff (20) in order to determine whether the speed of the hand region (21, 22, 23, 24) of the medical staff (20) lies in a predetermined speed range and furthermore to generate the control signal depending on whether the speed of the hand region (21, 22, 23, 24) of the medical staff (20) lies in the predetermined speed range.

4. Control unit (60) according to one of the preceding claims, wherein the control unit (60) is furthermore embodied to receive a positioning signal which represents a position or a change in the position of an adjustable operating table (30) or of an object with which the operation light (40) can collide, and to generate the control signal depending on the positioning signal.

5. Control unit (60) according to one of the preceding claims, wherein the control unit (60) is furthermore embodied to identify at least a movement of a head, a facial expression or an eye movement of medical staff (20) and to generate the control signal depending on the movement of the head, the facial expression or the eye movement.

6. Operation light system comprising a control unit according to one of the preceding claims and an operation light (40) for generating light, wherein the operation light comprises:
a camera (43) for acquiring an image of an operation field (14) and for generating an image signal;
an image signal output (44) for transmitting the image signal to a control unit (60);
a control signal input (42, 52, 55, 57, 59) for receiving a control signal from the control unit (60);
wherein the operation light (40) is embodied to modify at least a direction or a spectral property of the light, or a dimension or form of the region illuminated by the light, depending on the control signal.

7. Operation light system according to the preceding claim, in which the operation light (40) at least is arranged on a rail apparatus or comprises a plurality of individual lights which are arranged in an array or in a different uniform or nonuniform style.

8. Method for operating an operation light (40) for generating light, comprising the following steps:
acquiring (103) an image of the operation field (14) and generating an image signal by means of a camera (43);
transmitting (104) the image signal from the camera (43) to a control unit (60);
**characterized by** the following steps:
identifying a hand or a forearm of medical staff (20) in the image signal;
determining (106) a gesture of the hand or of the forearm (21) of the medical staff on the basis of the image signal,
generating (113) a control signal depending on the image signal and depending on the determined gesture;
controlling (114), by means of the control signal, at least a direction or a spectral property of the light, or a dimension or form of the region illuminated by the light.

9. Method according to the preceding claim, furthermore comprising the following step:
determining (107) whether the position of the hand region (21, 22, 23, 24) of the medical staff (20) lies in a predetermined spatial region (14),
wherein the control signal is furthermore generated depending on whether the position of the hand region (21, 22, 23, 24) lies in the predetermined spatial region.

10. Method according to either of Claims 8 and 9, furthermore comprising the following step:
determining (108) the speed of a hand region (21, 22, 23, 24) of the medical staff (20);
determining (109) whether the speed of the hand region (21, 22, 23, 24) of the medical staff (20) lies in a predetermined speed range (14),
wherein the control signal is furthermore generated depending on whether the speed of the hand region (21, 22, 23, 24) of the medical staff (20) lies in the predetermined speed range (14).

11. Method according to one of Claims 8 to 10, furthermore comprising the following step:
acquiring (111) a positioning signal which indicates a position or a change in the position of an operating table (30) or an object with which the operation light (40) can collide,
wherein the control signal is generated depending on the positioning signal.

12. Method according to one of Claims 8 to 11, furthermore comprising the following step:
acquiring (101) an identity of the medical staff (20) by means of a camera (43) or a sensor (49).

13. Method according to the preceding claim, furthermore comprising the following step:
logging (102) the medical staff (20) onto an operating system.

## Revendications

1. Unité de commande (60) destinée à commander une lampe opératoire (40) pour générer de la lumière, comprenant :
une entrée de signal d'image (61) destinée à recevoir un signal d'image en provenance d'une caméra (43) pour acquérir une image d'un champ opératoire (14) ;
une sortie de signal de commande (63) destinée à délivrer à la lampe opératoire (40) un signal de commande pour commander au moins soit une direction, soit une propriété spectrale de la lumière, soit une taille, soit une forme de la région éclairée par la lumière ;
**caractérisée en ce que**
l'unité de commande (60) est conçue pour reconnaître, sur la base du signal d'image, un geste d'une main ou d'un avant-bras (21) du personnel médical (20) et pour générer le signal de commande en fonction du geste.

2. Unité de commande (60) selon la revendication précédente, dans laquelle l'unité de commande (60) est en outre conçue pour déterminer, sur la base du signal d'image, si une position d'une région de la main (21, 22, 23, 24) du personnel médical (20) se trouve dans une région prédéterminée de l'espace (14), et pour générer en outre le signal de commande en fonction du fait que la position de la région de la main (21, 22, 23, 24) se trouve ou non dans la région prédéterminée de l'espace.

3. Unité de commande (60) selon l'une quelconque des revendications précédentes, dans laquelle l'unité de commande (60) est en outre conçue pour déterminer une vitesse d'une région de la main (21, 22, 23, 24) du personnel médical (20), pour déterminer si la vitesse de la région de la main (21, 22, 23, 24) du personnel médical (20) se situe dans un domaine de vitesse prédéterminé, et pour générer en outre le signal de commande en fonction du fait que la vitesse de la région de la main (21, 22, 23, 24) du personnel médical (20) se situe ou non dans le domaine de vitesse prédéterminé.

4. Unité de commande (60) selon l'une quelconque des revendications précédentes, dans laquelle l'unité de commande (60) est en outre conçue pour recevoir un signal de positionnement représentant une position ou un changement de position d'une table opératoire réglable (30) ou d'un objet avec lequel la lampe opératoire (40) peut entrer en collision, et pour générer le signal de commande en fonction du signal de positionnement.

5. Unité de commande (60) selon l'une quelconque des revendications précédentes, dans laquelle l'unité de commande (60) est en outre conçue pour reconnaître au moins soit un mouvement d'une tête, une expression faciale, soit un mouvement des yeux du personnel médical (20) et pour générer le signal de commande en fonction du mouvement de la tête, de l'expression faciale ou du mouvement des yeux.

6. Système de lampe opératoire comprenant une unité de commande selon l'une quelconque des revendications précédentes et une lampe opératoire (40) destinée à générer de la lumière, dans lequel la lampe opératoire comprend :
une caméra (43) destinée à acquérir une image d'un champ opératoire (14) et à générer un signal d'image ;
une sortie de signal d'image (44) destinée à délivrer le signal d'image à une unité de commande (60) ;
une entrée de signal de commande (42, 52, 55, 57, 59) destinée à recevoir un signal de commande de l'unité de commande (60) ;
dans lequel la lampe opératoire (40) est conçue pour modifier soit une direction, soit une propriété spectrale de la lumière, soit une taille, soit une forme de la région éclairée par la lumière en fonction du signal de commande.

7. Système de lampe opératoire selon la revendication précédente, dans lequel la lampe opératoire (40) est au moins disposée sur un dispositif à rails, ou comprend une pluralité de lampes individuelles qui sont agencées en une matrice ou d'une autre manière régulière ou irrégulière.

8. Procédé destiné à faire fonctionner une lampe opératoire (40) pour générer de la lumière, comprenant les étapes suivantes :
acquérir (103) une image du champ opératoire (14) et
générer un signal d'image au moyen d'une caméra (43) ;
transmettre (104) le signal d'image de la caméra (43) à une unité de commande (60) ;
**caractérisé par** les étapes suivantes :
reconnaître une main ou un avant-bras d'un personnel médical (20) dans le signal d'image ;
déterminer (106) un geste de la main ou de l'avant-bras (21) du personnel médical sur la base du signal d'image,
générer (113) un signal de commande en fonction du signal d'image et en fonction du geste déterminé ;
commander (114) au moins soit une direction, soit une propriété spectrale de la lumière, soit une taille, soit une forme de la région éclairée par la lumière au moyen du signal de commande.

9. Procédé selon la revendication précédente, comprenant en outre l'étape suivante :
déterminer (107) si la position de la région de la main (21, 22, 23, 24) du personnel médical (20) se trouve dans une région prédéterminée de l'espace (14),
dans lequel le signal de commande est en outre généré en fonction du fait que la position de la région de la main (21, 22, 23, 24) se situe ou non dans la région prédéterminée de l'espace.

10. Procédé selon l'une quelconque des revendications 8 et 9, comprenant en outre les étapes suivantes :
déterminer (108) la vitesse d'une région de la main (21, 22, 23, 24) du personnel médical (20);
déterminer (109) si la vitesse de la région de la main (21, 22, 23, 24) du personnel médical (20) se situe dans un domaine de vitesse prédéterminé (14),
dans lequel le signal de commande est en outre généré en fonction du fait que la vitesse de la région de la main (21, 22, 23, 24) du personnel médical (20) se situe ou non dans la région prédéterminée de l'espace (14).

11. Procédé selon l'une quelconque des revendications 8 à 10, comprenant en outre l'étape suivante :
acquérir (111) un signal de positionnement qui indique une position ou un changement de position d'une table opératoire (30) ou d'un objet avec lequel la lampe opératoire (40) peut entrer en collision,
dans lequel le signal de commande est généré en fonction du signal de positionnement.

12. Procédé selon l'une quelconque des revendications 8 à 11, comprenant en outre l'étape suivante :
acquérir (101) une identité du personnel médical (20) au moyen d'une caméra (43) ou d'un capteur (49).

13. Procédé selon l'une quelconque des revendications précédentes, comprenant en outre l'étape suivante :
inscrire (102) le personnel médical (20) à un système opératoire.
